# EUROPEAN PATENT SPECIFICATION

(11) **EP 3 096 786 B1**
(45) Date of publication and mention of the grant of the patent: **07.07.2021**
(21) Application number: 15708581.2
(22) Date of filing: 15.01.2015
(51) Int. Cl.: A61K 39/09, A61K 39/385, A61K 39/00, A61K 39/39, A61P 11/00, A61P 31/04

(54) **STREPTOCOCCUS PNEUMONIAE CAPSULAR POLYSACCHARIDES AND CONJUGATES THEREOF**
KAPSULARE POLYSACCHARIDE AUS STREPTOCOCCUS PNEUMONIAE UND KONJUGATE DAVON
POLYSACCHARIDES CAPSULAIRES DE STREPTOCOCCUS PNEUMONIAE ET LEURS CONJUGUÉS

(30) Priority: 21.01.2014 US 201461929561 P
(43) Date of publication of application: 30.11.2016
(60) Divisional application: 19179577.2
(73) Proprietor: Pfizer Inc., New York, NY 10017 (US)
(72) Inventor: HAN, Mingming, Nazareth, Pennsylvania 18064 (US); PRASAD, Avvari Krishna, Chapel Hill, North Carolina 27516 (US); COOPER, David, Monroe, New York 10950 (US); WATSON, Wendy Jo, Blue Bell, Pennsylvania 19422 (US)
(74) Representative: Pfizer
(86) International application number: PCT/IB2015/050316
(87) International publication number: WO 2015/110942

(56) References cited:
- EP-A2- 0 157 899
- EP-A2- 2 384 765
- WO-A1-2008/157590
- WO-A1-2012/119972
- WO-A1-2012/119972
- WO-A1-2012/158701
- WO-A1-2012/158701
- WO-A1-2014/027302
- WO-A1-2014/027302
- WO-A2-2007/071707
- WO-A2-2007/071707
- WO-A2-2011/110531
- WO-A2-2014/097099
- WO-A2-2014/097099
- US-A- 5 623 057
- US-A- 5 714 354
- US-A1- 2013 273 098
- BUSSAT B ET AL: "Molecular size characterization of bacterial capsular polysaccharide vaccines by high performance liquid chromatography", BIOLOGICALS, ACADEMIC PRESS LTD., LONDON, GB, vol. 18, no. 2, 1 April 1990 (1990-04-01), pages 117-121, XP022954054, ISSN: 1045-1056, DOI: 10.1016/1045-1056(90)90021-Q [retrieved on 1990-04-01]
- PER-ERIK JANSSON ET AL: "Structural studies of the capsular polysaccharide from Streptococcus pneumoniae types 15B and 15C", CARBOHYDRATE RESEARCH, vol. 162, no. 1, 1 April 1987 (1987-04-01) , pages 111-116, XP055210747, ISSN: 0008-6215, DOI: 10.1016/0008-6215(87)80205-7
- J G HOWARD ET AL: "Studies on Immunological Paralysis: V. THE INFLUENCE OF MOLECULAR WEIGHT ON THE IMMUNOGENICITY, TOLEROGENICITY AND ANTIBODY-NEUTRALIZING ACTIVITY OF TYPE III PNEUMOCOCCAL POLYSACCHARIDE", IMMUNOLOGY., vol. 21, no. 3, 1 January 1971 (1971-01-01), pages 535-546, XP55210763, GB ISSN: 0019-2805

### SEQUENCE LISTING

<110> Pfizer Inc.
<120> Streptococcus pneumoniae capsular polysaccharides and conjugates
   thereof
<130> PC72026A
<150> US 61/929,561
   <151> 2014-01-21
<160> 40
<170> PatentIn version 3.5
<210> 1
   <211> 21
   <212> DNA
   <213> Artificial Sequence
<220>
   <223> "A class" CpG oligonucleotide
<400> 1
   ggggacgacg tcgtgggggg g 21
<210> 2
   <211> 21
   <212> DNA
   <213> Artificial Sequence
<220>
   <223> "A class" CpG oligonucleotide
<400> 2
   ggggacgacg tcgtgggggg g 21
<210> 3
   <211> 21
   <212> DNA
   <213> Artificial Sequence
<220>
   <223> "B class" CpG oligonucleotide
<400> 3
   tcgtcgtttt tcggtgcttt t 21
<210> 4
   <211> 21
   <212> DNA
   <213> Artificial Sequence
<220>
   <223> "B class" CpG oligonucleotide
<400> 4
   tcgtcgtttt tcggtcgttt t 21
<210> 5
   <211> 24
   <212> DNA
   <213> Artificial Sequence
<220>
   <223> "B class" CpG oligonucleotide
<400> 5
   tcgtcgtttt gtcgttttgt cgtt 24
<210> 6
   <211> 24
   <212> DNA
   <213> Artificial Sequence
<220>
   <223> "B class" CpG oligonucleotide
<400> 6
   tcgtcgtttc gtcgttttgt cgtt 24
<210> 7
   <211> 24
   <212> DNA
   <213> Artificial Sequence
<220>
   <223> "B class" CpG oligonucleotide
<400> 7
   tcgtcgtttt gtcgtttttt tcga 24
<210> 8
   <211> 21
   <212> DNA
   <213> Artificial Sequence
<220>
   <223> B-Class oligonucleotide
<400> 8
   tcgtcgtttt tcggtgcttt t 21
<210> 9
   <211> 21
   <212> DNA
   <213> Artificial Sequence
<220>
   <223> B-Class oligonucleotide
<400> 9
   tcgtcgtttt tcggtcgttt t 21
<210> 10
   <211> 24
   <212> DNA
   <213> Artificial Sequence
<220>
   <223> B-Class oligonucleotide
<400> 10
   tcgtcgtttt gtcgttttgt cgtt 24
<210> 11
   <211> 24
   <212> DNA
   <213> Artificial Sequence
<220>
   <223> B-Class oligonucleotide
<400> 11
   tcgtcgtttc gtcgttttgt cgtt 24
<210> 12
   <211> 24
   <212> DNA
   <213> Artificial Sequence
<220>
   <223> B-Class oligonucleotide
<400> 12
   tcgtcgtttt gtcgtttttt tcga 24
<210> 13
   <211> 22
   <212> DNA
   <213> Artificial Sequence
<220>
   <223> C class CpG Oligonucleotide
<400> 13
   tcgcgtcgtt cggcgcgcgc cg 22
<210> 14
   <211> 23
   <212> DNA
   <213> Artificial Sequence
<220>
   <223> C class CpG Oligonucleotide
<400> 14
   tcgtcgacgt tcggcgcgcg ccg 23
<210> 15
   <211> 21
   <212> DNA
   <213> Artificial Sequence
<220>
   <223> C class CpG Oligonucleotide
<400> 15
   tcggacgttc ggcgcgcgcc g 21
<210> 16
   <211> 19
   <212> DNA
   <213> Artificial Sequence
<220>
   <223> C class CpG Oligonucleotide
<400> 16
   tcggacgttc ggcgcgccg 19
<210> 17
   <211> 20
   <212> DNA
   <213> Artificial Sequence
<220>
   <223> C class CpG Oligonucleotide
<400> 17
   tcgcgtcgtt cggcgcgccg 20
<210> 18
   <211> 20
   <212> DNA
   <213> Artificial Sequence
<220>
   <223> C class CpG Oligonucleotide
<400> 18
   tcgacgttcg gcgcgcgccg 20
<210> 19
   <211> 18
   <212> DNA
   <213> Artificial Sequence
<220>
   <223> C class CpG Oligonucleotide
<400> 19
   tcgacgttcg gcgcgccg 18
<210> 20
   <211> 18
   <212> DNA
   <213> Artificial Sequence
<220>
   <223> C class CpG Oligonucleotide
<400> 20
   tcgcgtcgtt cggcgccg 18
<210> 21
   <211> 22
   <212> DNA
   <213> Artificial Sequence
<220>
   <223> C class CpG Oligonucleotide
<400> 21
   tcgcgacgtt cggcgcgcgc cg 22
<210> 22
   <211> 22
   <212> DNA
   <213> Artificial Sequence
<220>
   <223> C class CpG Oligonucleotide
<400> 22
   tcgtcgtttt cggcgcgcgc cg 22
<210> 23
   <211> 22
   <212> DNA
   <213> Artificial Sequence
<220>
   <223> C class CpG Oligonucleotide
<400> 23
   tcgtcgtttt cggcggccgc cg 22
<210> 24
   <211> 24
   <212> DNA
   <213> Artificial Sequence
<220>
   <223> C class CpG Oligonucleotide
<400> 24
   tcgtcgtttt acggcgccgt gccg 24
<210> 25
   <211> 23
   <212> DNA
   <213> Artificial Sequence
<220>
   <223> C class CpG Oligonucleotide
<400> 25
   tcgtcgtttt cggcgcgcgc cgt 23
<210> 26
   <211> 22
   <212> DNA
   <213> Artificial Sequence
<220>
   <223> C-Class oligonucleotides
<400> 26
   tcgcgtcgtt cggcgcgcgc cg 22
<210> 27
   <211> 23
   <212> DNA
   <213> Artificial Sequence
<220>
   <223> C-Class oligonucleotide
<400> 27
   tcgtcgacgt tcggcgcgcg ccg 23
<210> 28
   <211> 21
   <212> DNA
   <213> Artificial Sequence
<220>
   <223> C-Class oligonucleotide
<400> 28
   tcggacgttc ggcgcgcgcc g 21
<210> 29
   <211> 19
   <212> DNA
   <213> Artificial Sequence
<220>
   <223> C-Class oligonucleotide
<400> 29
   tcggacgttc ggcgcgccg 19
<210> 30
   <211> 20
   <212> DNA
   <213> Artificial Sequence
<220>
   <223> C-Class oligonucleotide
<400> 30
   tcgcgtcgtt cggcgcgccg 20
<210> 31
   <211> 20
   <212> DNA
   <213> Artificial Sequence
<220>
   <223> C-Class oligonucleotide
<400> 31
   tcgacgttcg gcgcgcgccg 20
<210> 32
   <211> 18
   <212> DNA
   <213> Artificial Sequence
<220>
   <223> C-Class oligonucleotide
<400> 32
   tcgacgttcg gcgcgccg 18
<210> 33
   <211> 18
   <212> DNA
   <213> Artificial Sequence
<220>
   <223> C-Class oligonucleotide
<400> 33
   tcgcgtcgtt cggcgccg 18
<210> 34
   <211> 22
   <212> DNA
   <213> Artificial Sequence
<220>
   <223> C-Class oligonucleotide
<400> 34
   tcgcgacgtt cggcgcgcgc cg 22
<210> 35
   <211> 22
   <212> DNA
   <213> Artificial Sequence
<220>
   <223> C-Class oligonucleotide
<400> 35
   tcgtcgtttt cggcgcgcgc cg 22
<210> 36
   <211> 22
   <212> DNA
   <213> Artificial Sequence
<220>
   <223> C-Class oligonucleotide
<400> 36
   tcgtcgtttt cggcggccgc cg 22
<210> 37
   <211> 24
   <212> DNA
   <213> Artificial Sequence
<220>
   <223> C-Class oligonucleotide
<400> 37
   tcgtcgtttt acggcgccgt gccg 24
<210> 38
   <211> 23
   <212> DNA
   <213> Artificial Sequence
<220>
   <223> C-Class oligonucleotide
<400> 38
   tcgtcgtttt cggcgcgcgc cgt 23
<210> 39
   <211> 23
   <212> DNA
   <213> Artificial Sequence
<220>
   <223> P class CpG oligonucleotide
<400> 39
   tcgtcgacga tcggcgcgcg ccg 23
<210> 40
   <211> 23
   <212> DNA
   <213> Artificial Sequence
<220>
   <223> P class CpG oligonucleotide
<400> 40
   tcgtcgacga tcggcgcgcg ccg 23

## Description

### Field of the invention

The invention relates to processes for the preparation of immunogenic conjugates comprising *Streptococcus pneumoniae* serotype 15B capsular polysaccharide covalently linked to a carrier protein, conjugates obtained by such processes and immunogenic compositions and vaccines comprising them.

### Background

*Streptococcus pneumoniae* are Gram-positive, lancet shaped cocci that are usually seen in pairs (diplococci), but also in short chains or as single cells. They grow readily on blood agar plates with glistening colonies and display alpha hemolysis unless grown anaerobically where they show beta hemolysis. The cells of most pneumococcal serotypes have a capsule which is a polysaccharide coating surrounding each cell. This capsule is a determinant of virulence in humans, as it interferes with phagocytosis by preventing antibodies from attaching to the bacterial cells. Currently there are more than 90 known pneumococcal capsular serotypes identified, with the 23 most common serotypes accounting for approximately 90% of invasive disease worldwide. As a vaccine, the pneumococcal polysaccharide coat can confer a reasonable degree of immunity to *Streptococcus pneumoniae* in individuals with developed or unimpaired immune systems, but the capsular polysaccharide conjugated to a suitable carrier protein allows for an immune response in infants and elderly who are also at most risk for pneumococcal infections.

Since the introduction of the first 7-valent pneumococcal conjugate vaccine (PCV7 or Prevnar) in 2000, invasive disease from those seven serotypes (4, 6B, 9V, 14, 18C, 19F, and 23F) has nearly disappeared. The addition of serotypes 1, 3, 5, 6A, 7F and 19A in Prevnar 13 further decreased the numbers of invasive pneumococcal disease.

However, the incidence of invasive pneumococcal diseases caused by non-vaccine serotypes such as *Streptococcus pneumoniae* serotypes 15A, 15B and 15C has recently increased (see for example Beall B. et al, Journal of Clinical Microbiology. 44(3):999-1017, 2006, or Jacobs et Al, Clin Infect Dis. (2008) 47 (11): 1388-1395). None of the currently marketed pneumococcal vaccine provides an appropriate protection against serotype 15B *Streptococcus pneumoniae* in human and in particular in children less than 2 years old. Therefore, there is a need for immunogenic compositions that can be used to induce an immune response against serotype 15B *Streptococcus pneumoniae.* It would also be an additional benefit if such immunogenic composition could be used to protect subjects against serotype 15C and/or 15A *Streptococcus pneumoniae.*

### Summary of the invention

The invention relates to the embodiments characterized by the claims.

In particular, the invention relates to a process for the preparation of an immunogenic conjugate comprising *Streptococcus pneumoniae* serotype 15B capsular polysaccharide covalently linked to a carrier protein, the process comprising the steps of:
(a) compounding an activated serotype 15B capsular polysaccharide with a carrier protein, wherein said activated polysaccharide is obtained by a process comprising the steps of reacting an isolated *Streptococcus pneumoniae* serotype 15B capsular polysaccharide comprising at least 0.6 mM acetate per mM of said serotype 15B capsular polysaccharide, with an oxidizing agent, wherein said activated polysaccharide has a molecular weight between 100 and 300kDa; and
(b) reacting the compounded, activated polysaccharide and carrier protein with a reducing agent to form a serotype 15B capsular polysaccharide-carrier protein conjugate;
   wherein step (a) and step (b) are carried out in DMSO.

The invention also relates to an immunogenic conjugate obtained by the process of the invention and to an immunogenic composition comprising said immunogenic conjugate and a physiologically acceptable vehicle.

The invention further relates to vaccine comprising said immunogenic composition and to the use of said vaccine and immunogenic composition for use in a method of protecting a subject against an infection with serotype 15B Streptococcus pneumoniae or in a method of treating or preventing a Streptococcus pneumoniae infection, disease or condition associated with serotype 15B Streptococcus pneumoniae in a subject, said method comprising the step of administering a therapeutically or prophylactically effective amount of immunogenic composition or of said vaccine.

In one aspect the present disclosure provides an isolated *Streptococcus pneumoniae* serotype 15B capsular polysaccharide having a molecular weight between 5kDa and 500kDa.

### Brief description of the drawings

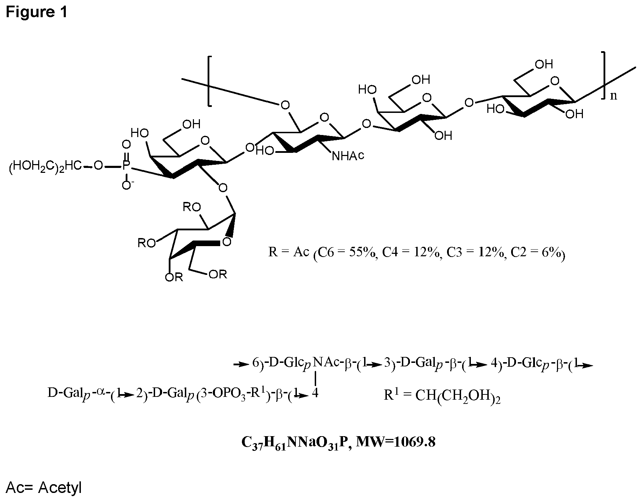

Figure 1 - Structure of Pneumococcal Capsular polysaccharide Serotype 15B Repeat Unit

### Detailed description of the invention

The present invention may be understood more readily by reference to the following detailed description of the preferred embodiments of the invention and the Examples included herein. Unless defined otherwise, all technical and scientific terms used herein have the same meaning as commonly understood by one of ordinary skill in the art to which the invention pertains. In describing the embodiments and claiming the invention, certain terminology will be used in accordance with the definitions set out below.

### Definitions

As used herein, the "molecular weight" of a polysaccharide or of a polysaccharide-carrier protein conjugate refers to molecular weight calculated by size exclusion chromatography (SEC) combined with multiangle laser light scattering detector (MALLS).

As used herein, the term "free polysaccharide" means a serotype 15B capsular polysaccharide that is not covalently conjugated to the carrier protein, but is nevertheless present in the serotype 15B capsular polysaccharide-carrier protein conjugate composition. The free polysaccharide may be non-covalently associated with (i.e., non-covalently bound to, adsorbed to, or entrapped in or with) the polysaccharide-carrier protein conjugate.
The percentage of free polysaccharide is measured after the final purification of the serotype 15B capsular polysaccharide-carrier protein conjugate. Preferably it is measured within 4 weeks after the final purification. It is expressed as a percentage of the total polysaccharide in the sample.

As used herein, the term "serotype 15B polysaccharide" or "serotype 15B capsular polysaccharide" refers to a *Streptococcus pneumoniae* serotype 15B capsular polysaccharide.

As used herein, the term "serotype 15B glycoconjugate" or "serotype 15B conjugate" refers to an isolated serotype 15B polysaccharide covalently conjugated to a carrier protein.

As used herein, the term "degree of oxidation" (DO) refers to the number of sugar repeat units per aldehyde group generated when the isolated polysaccharide is activated with an oxidizing agent. The degree of oxidation of a polysaccharide can be determined using routine methods known to the man skilled in the art.

As used herein, the term "subject" refers to a mammal, including a human, or to a bird, fish, reptile, amphibian or any other animal. The term "subject" also includes household pets or research animals. Non-limiting examples of household pets and research animals include: dogs, cats, pigs, rabbits, rats, mice, gerbils, hamsters, guinea pigs, ferrets, monkeys, birds, snakes, lizards, fish, turtles, and frogs. The term "subject" also includes livestock animals. Non-limiting examples of livestock animals include: alpaca, bison, camel, cattle, deer, pigs, horses, llamas, mules, donkeys, sheep, goats, rabbits, reindeer, yak, chickens, geese, and turkeys.

### Isolated serotype 15B capsular polysaccharide

Isolated 15B capsular polysaccharides are not part of the claimed subject matter. As shown in figure 1, the polysaccharide repeating unit of serotype 15B consists of a branched trisaccharide backbone (one N-acetylglucosamine (Glc*ₚ*NAc), one galactopyranose (Gal*ₚ*) and one glucopyranose (Glc*ₚ*)) with an αGal*ₚ*-βGal*ₚ* disaccharide branch linked to the C4 hydroxyl group of Glc*ₚ*NAc. The phosphoglycerol is linked to the C3 hydroxyl group of the βGal*ₚ* residue in the disaccharide branch. Serotype 15B capsular polysaccharide is O-acetylated and the total amount of O-acetylation is approximately 0.8 to 0.9 O-acetyl groups per polysaccharide repeating unit (see for example C. Jones et Al, Carbohydrate Research, 340 (2005) 403-409). Capsular polysaccharide from serotype 15C serotype has the identical backbone structure as serotype 15B but lacks the O-acetylation.

The isolated serotype 15B polysaccharide of the disclosure can be obtained by a process comprising the steps of:
(a) preparing a fermentation culture of serotype 15B *Streptococcus pneumonia* bacterial cells;
(b) lysing the bacterial cells in said fermentation culture;
(c) purifying serotype 15B polysaccharide from the fermentation culture; and,
(d) sizing the purified serotype 15B polysaccharide by high pressure homogenization.

Serotype 15B polysaccharides can be obtained directly from bacteria using isolation procedures known to one of ordinary skill in the art (see for example methods disclosed U.S. Patent App. Pub. Nos. 20060228380, 20060228381, 20070184071, 20070184072, 20070231340, and 20080102498 or WO2008118752). In addition, they can be produced using synthetic protocols.

Serotype 15B *Streptococcus pneumoniae* strains may be obtained from established culture collections (such as for example ATCC deposit strain No ATCC10354 or strain available from the Streptococcal Reference Laboratory of the Center for disease control and prevention, Atlanta, GA)) or clinical specimens.

The bacterial cells are preferably grown in a soy based medium. Following fermentation of bacterial cells that produce *Streptococcus pneumoniae* serotype 15B capsular polysaccharides, the bacterial cells are lysed to produce a cell lysate. The bacterial cells may be lysed using any lytic agent. A "lytic agent" is any agent that aids in cell wall breakdown and release of autolysin which causes cellular lysis including, for example, detergents. As used herein, the term "detergent" refers to any anionic or cationic detergent capable of inducing lysis of bacterial cells. Representative examples of such detergents for use within the methods of the present invention include deoxycholate sodium (DOC), N-lauroyl sarcosine, chenodeoxycholic acid sodium, and saponins.

In one embodiment of the present disclosure, the lytic agent used for lysing bacterial cells is DOC. DOC is the sodium salt of the bile acid deoxycholic acid, which is commonly derived from biological sources such as cows or oxen. DOC activates the LytA protein, which is an autolysin that is involved in cell wall growth and division in *Streptococcus pneumoniae.* The LytA protein has choline binding domains in its C-terminal portion, and mutations of the lytA gene are known to produce LytA mutants that are resistant to lysis with DOC.

In one embodiment of the present disclosure, the lytic agent used for lysing bacterial cells is a non-animal derived lytic agent. Non-animal derived lytic agents for use within the methods of the present disclosure include agents from non-animal sources with modes of action similar to that of DOC (i. e., that affect LytA function and result in lysis of *Streptococcus pneumoniae* cells). Such non-animal derived lytic agents include, but are not limited to, analogs of DOC, surfactants, detergents, and structural analogs of choline. In one embodiment, the non-animal derived lytic agent is selected from the group consisting of decanesulfonic acid, tert-octylphenoxy poly(oxyethylene)ethanols (e.g. Igepal® CA-630, CAS #: 9002-93-1, available from Sigma Aldrich, St. Louis, MO), octylphenol ethylene oxide condensates (e.g. Triton® X-100, available from Sigma Aldrich, St. Louis, MO), N-lauroyl sarcosine, N-lauroyl sarcosine sodium, lauryl iminodipropionate, sodium dodecyl sulfate, chenodeoxycholate, hyodeoxycholate, glycodeoxycholate, taurodeoxycholate, taurochenodeoxycholate, and cholate. In another embodiment, the non-animal derived lytic agent is N-lauroyl sarcosine. In another embodiment, the lytic agent is N-lauroyl sarcosine sodium.

The serotype 15B polysaccharide may then be isolated from the cell lysate using purification techniques known in the art, including the use of centrifugation, depth filtration, precipitation, ultra-filtration, treatment with activate carbon, diafiltration and/or column chromatography (See, for example, U.S. Patent App. Pub. Nos. 20060228380, 20060228381, 20070184071, 20070184072, 20070231340, and 20080102498 or WO2008118752). The purified serotype 15B capsular polysaccharide can then be used for the preparation of immunogenic conjugates.

Preferably, in order to generate conjugates with advantageous filterability characteristics and/or yields, sizing of the polysaccharide to a lower molecular weight (MW) range is performed prior to the conjugation to a carrier protein. Advantageously, the size of the purified serotype 15B polysaccharide is reduced while preserving critical features of the structure of the polysaccharide such as for example the presence of O-acetyl groups. Preferably, the size of the purified serotype 15B polysaccharide is reduced by mechanical homogenization.

In a preferred embodiment, the size of the purified serotype 15B polysaccharide is reduced by high pressure homogenization. High pressure homogenization achieves high shear rates by pumping the process stream through a flow path with sufficiently small dimensions. The shear rate is increased by using a larger applied homogenization pressure and exposure time can be increased by recirculating the feed stream through the homogenizer.

The high pressure homogenization process is particularly appropriate for reducing the size of the purified serotype 15B polysaccharide while preserving the structural features of the polysaccharide such as the presence of O-acetyl groups.

The isolated serotype 15B capsular polysaccharide obtained by purification of serotype 15B polysaccharide from the *Streptococcus pneumoniae* lysate and optionally sizing of the purified polysaccharide can be characterized by different parameters including for example the molecular weight, the mM of glycerol per mM of said serotype 15B capsular polysaccharide or the mM of acetate per mM of said serotype 15B capsular polysaccharide.

The degree of O-acetylation of the polysaccharide can be determined by any method known in the art, for example, by proton NMR (see for example Lemercinier and Jones (1996) Carbohydrate Research 296; 83-96, Jones and Lemercinier (2002) J. Pharmaceutical and Biomedical Analysis 30; 1233-1247, WO 05/033148 or WO00/56357). Another commonly used method is described in Hestrin (1949) J. Biol. Chem. 180; 249-261. Preferably, the presence of O-acetyl groups is determined by ion-HPLC analysis.

The presence of O-acetyl in a purified, isolated or activated serotype 15B capsular polysaccharide or in a serotype 15B polysaccharide-carrier protein conjugate is expressed as the number of mM of acetate per mM of said polysaccharide or as the number of O-acetyl group per polysaccharide repeating unit.

The presence of glycerolphosphate side chains can be determined by measurement of glycerol using high performance anion exchange chromatography with pulsed amperometric detection (HPAEC-PAD) after its release by treatment of the polysaccharide with hydrofluoric acid (HF). The presence of glycerol in a purified, isolated or activated serotype 15B polysaccharide or in a serotype 15B polysaccharide-carrier protein conjugate is expressed as the number of mM of glycerol per mM of serotype 15B polysaccharide.

The isolated serotype 15B capsular polysaccharide can also be produced synthetically using methods known to the man skilled in the art.

In a preferred embodiment, of the disclosure the isolated serotype 15B capsular polysaccharide has a molecular weight between 5 and 500kDa, 50 and 500 kDa, 50 and 450kDa, 100 and 400kDa, 100 and 350 kDa. In a preferred embodiment, of the disclosure the isolated serotype 15B capsular polysaccharide has a molecular weight between 100 and 350kDa. In a preferred embodiment, of the disclosure the isolated serotype 15B capsular polysaccharide has a molecular weight between 100 and 300kDa. In a preferred embodiment, of the disclosure the isolated serotype 15B capsular polysaccharide has a molecular weight between 150 and 300kDa.

In a preferred embodiment, of the disclosure the isolated serotype 15B capsular polysaccharide comprises at least 0.1, 0.2, 0.3, 0.4, 0.5, 0.6, 0.7 or 0.8 mM acetate per mM of said serotype 15B capsular polysaccharide. In a preferred embodiment, of the disclosure the isolated serotype 15B capsular polysaccharide comprises at least 0.5, 0.6 or 0.7 mM acetate per mM of said serotype 15B capsular polysaccharide. In a preferred embodiment, of the disclosure the isolated serotype 15B capsular polysaccharide comprises at least 0.6 mM acetate per mM of said serotype 15B capsular polysaccharide. In a preferred embodiment of the disclosure the isolated serotype 15B capsular polysaccharide comprises at least 0.7 mM acetate per mM of said serotype 15B capsular polysaccharide. In a preferred embodiment, of the disclosure, the presence of O-acetyl groups is determined by ion-HPLC analysis.

In a preferred embodiment, of the disclosure the isolated serotype 15B capsular polysaccharide comprises at least 0.1, 0.2, 0.3, 0.4, 0.5, 0.6, 0.7 or 0.8 mM glycerol per mM of said serotype 15B capsular polysaccharide. In a preferred embodiment of the disclosure, the isolated serotype 15B capsular polysaccharide comprises at least 0.5, 0.6 or 0.7 mM glycerol per mM of said serotype 15B capsular polysaccharide. In a preferred embodiment of the disclosure the isolated serotype 15B capsular polysaccharide comprises at least 0.6 mM glycerol per mM of said serotype 15B capsular polysaccharide. In a preferred embodiment, of the disclosure the isolated serotype 15B capsular polysaccharide comprises at least 0.7 mM glycerol per mM of said serotype 15B capsular polysaccharide.

In a preferred embodiment, the isolated serotype 15B capsular polysaccharide has a molecular weight between 100 and 350 kDa, preferably 150 and 350kDa, and comprises at least 0.6 mM acetate per mM of said serotype 15B capsular polysaccharide.

In a preferred embodiment, of the disclosure the isolated serotype 15B capsular polysaccharide has a molecular weight between 100 and 350 kDa, preferably 150 and 350kDa, and comprises at least 0.6 mM glycerol per mM of said serotype 15B capsular polysaccharide.

In a preferred embodiment, of the disclosure the isolated serotype 15B capsular polysaccharide comprises at least 0.6 mM acetate per mM of said serotype 15B capsular polysaccharide and at least 0.6 mM glycerol per mM of said serotype 15B capsular polysaccharide.

In a preferred embodiment, of the disclosure the isolated serotype 15B capsular polysaccharide has a molecular weight between 100 and 350 kDa, preferably 150 and 350kDa, and comprises at least 0.6 mM acetate per mM of said serotype 15B capsular polysaccharide and at least 0.6 mM glycerol per mM of said serotype 15B capsular polysaccharide.

### Serotype 15B capsular polysaccharide-carrier protein conjugate

The isolated serotype 15B capsular polysaccharide may be conjugated to a carrier protein to obtain an immunogenic conjugate. The isolated polysaccharide can be conjugated to the carrier protein by methods known to the skilled person (See, for example, U.S. Patent App. Pub. Nos. 20060228380, 20070184071, 20070184072, 20070231340, WO2012/119972 or WO2011/100151).

In an embodiment, of the disclosure the polysaccharide may be activated with 1-cyano-4-dimethylamino pyridinium tetrafluoroborate (CDAP) to form a cyanate ester. The activated polysaccharide may be coupled directly or via a spacer (linker) group to an amino group on the carrier protein. For example, the spacer could be cystamine or cysteamine to give a thiolated polysaccharide which could be coupled to the carrier via a thioether linkage obtained after reaction with a maleimide-activated carrier protein (for example using GMBS) or a haloacetylated carrier protein (for example using iodoacetimide or N-succinimidyl bromoacetate or SIAB, or SIA, or SBAP). Preferably, the cyanate ester (optionally made by CDAP chemistry) is coupled with hexane diamine or adipic acid dihydrazide (ADH) and the amino-derivatised saccharide is conjugated to the carrier protein using carbodiimide (e.g. EDAC or EDC) chemistry via a carboxyl group on the protein carrier. Such conjugates are described for example in WO93/15760, WO 95/08348 and WO 96129094.

Other suitable techniques use carbodiimides, hydrazides, active esters, norborane, p-nitrobenzoic acid, N-hydroxysuccinimide, S--NHS, EDC, TSTU. Many are described in International Patent Application Publication No. WO 98/42721. Conjugation may involve a carbonyl linker which may be formed by reaction of a free hydroxyl group of the saccharide with CDI (See Bethell et al., 1979,1. Biol. Chern. 254:2572-4; Hearn et al., 1981, J. Chromatogr.218:509-18) followed by reaction with a protein to form a carbamate linkage. This may involve reduction of the anomeric terminus to a primary hydroxyl group, optional protection/deprotection, of the primary hydroxyl group reaction of the primary hydroxyl group with CDI to form a CDI carbamate intermediate and coupling the CDI carbamate intermediate with an amino group on a protein.

In a preferred embodiment, of the disclosure the isolated serotype 15B capsular polysaccharide is conjugated to the carrier protein by reductive amination. Reductive amination involves activation of the polysaccharide by oxidation and conjugation of the activated polysaccharide to a protein carrier by reduction.

### Activation of serotype 15B capsular polysaccharide

An activated serotype 15B capsular polysaccharide is obtained by reacting an isolated serotype 15B capsular polysaccharide with an oxidizing agent. For example, said activated serotype 15B capsular polysaccharide can be obtained by a process comprising the following steps:
(a) preparing a fermentation culture of serotype 15B *Streptococcus pneumonia* bacterial cells;
(b) lysing the bacterial cells in said fermentation culture;
(c) purifying serotype 15B polysaccharide from the fermentation culture;
(d) sizing the purified serotype 15B polysaccharide by high pressure homogenization.
(e) reacting the sized serotype 15B polysaccharide with an oxidizing agent.

In a preferred embodiment, the concentration of isolated serotype 15B capsular polysaccharide which is reacted with an oxidizing agent is between 0.1 and 10 mg/mL, 0.5 and 5 mg/mL, 1 and 3 mg/mL, or about 2 mg/mL.

In a preferred embodiment, the oxidizing agent is periodate. The periodate oxidises vicinal hydroxyl groups to form carbonyl or aldehyde groups and causes cleavage of a C-C bond. The term 'periodate' includes both periodate and periodic acid. This term also includes both metaperiodate (IO₄⁻) and orthoperiodate (IO₆⁵⁻). The term 'periodate' also includes the various salts of periodate including sodium periodate and potassium periodate. In a preferred embodiment, the oxidizing agent is sodium periodate. In a preferred embodiment the periodate used for the oxidation of serotype 15B capsular polysaccharide is metaperiodate. In a preferred embodiment the periodate used for the oxidation of serotype 15B capsular polysaccharide is sodium metaperiodate.

In a preferred embodiment, the polysaccharide is reacted with 0.01 to 10, 0.05 to 5, 0.1 to 1, 0.5 to 1, 0.7 to 0.8, 0.05 to 0.5, 0.1 to 0.3 molar equivalent of oxidizing agent. In a preferred embodiment, the polysaccharide is reacted with about 0.1, 0.15, 0.2, 0.25, 0.3, 0.35, 0.4, 0.45, 0.5, 0.55, 0.6, 0.65, 0.7, 0.75, 0.8, 0.85, 0.9, 0.95 molar equivalent of oxidizing agent. In a preferred embodiment, the polysaccharide is reacted with about 0.15 molar equivalent of oxidizing agent. In a preferred embodiment, the polysaccharide is reacted with about 0.25 molar equivalent of oxidizing agent. In a preferred embodiment, the polysaccharide is reacted with about 0.5 molar equivalent of oxidizing agent. In a preferred embodiment, the polysaccharide is reacted with about 0.6 molar equivalent of oxidizing agent. In a preferred embodiment, the polysaccharide is reacted with about 0.7 molar equivalent of oxidizing agent.

In a preferred embodiment, the duration of the reaction is between 1 and 50, 10 and 30, 15 and 20, 15 and 17 hours or about 16 hours.

In a preferred embodiment, the temperature of the reaction is maintained between 15 and 45°C, 15 and 30°C, 20 and 25°C. In a preferred embodiment, the temperature of the reaction is maintained at about 23°C.

In a preferred embodiment, the oxidation reaction is carried out in a buffer selected from sodium phosphate, potassium phosphate, 2-(N-morpholino)ethanesulfonic acid (MES) or Bis-Tris. In a preferred embodiment, the buffer is potassium phosphate.

In a preferred embodiment, the buffer has a concentration of between 1 and 500 mM, 1 and 300mM, 50 and 200mM. In a preferred embodiment the buffer has a concentration of about 100mM.

In a preferred embodiment, the oxidation reaction is carried out at a pH between 4 and 8, 5 and 7, 5.5 and 6.5. In a preferred embodiment, the pH is about 6.

In preferred embodiment, the activated serotype 15B capsular polysaccharide is obtained by reacting 0.5 to 5 mg/mL of isolated serotype 15B capsular polysaccharide with 0.2 to 0.3 molar equivalent of periodate at a temperature between 20 and 25°C.

In a preferred embodiment, the activated serotype 15B capsular polysaccharide is purified. The activated serotype 15B capsular polysaccharide is purified according to methods known to the man skilled in the art such as gel permeation chromatography (GPC), dialysis or ultrafiltration/diafiltration. For example, the activated capsular polysaccharide is purified by concentration and diafiltration using an ultrafiltration device.

In a preferred embodiment, the disclosure relates to an activated serotype 15B capsular polysaccharide obtained or obtainable by the above disclosed process.

In a preferred embodiment, the degree of oxidation of the activated serotype 15B capsular polysaccharide is between 2 and 20, 2 and 15, 2 and 10, 2 and 5, 5 and 20, 5 and 15, 5 and 10, 10 and 20, 10 and 15, 15 and 20. In a preferred embodiment the degree of oxidation of the activated serotype 15B capsular polysaccharide is between 2 and 10, 4 and 8, 4 and 6, 6 and 8, 6 and 12, 8 and 12, 9 and 11, 10 and 16, 12 and 16, 14 and 18, 16 and 20, 16 and 18, or 18 and 20.

In a preferred embodiment, the activated serotype 15B capsular polysaccharide has a molecular weight between 100 and 300kDa. In a preferred embodiment, the activated serotype 15B capsular polysaccharide has a molecular weight between 150 and 300kDa. In a preferred embodiment, the activated serotype 15B capsular polysaccharide has a molecular weight between 100 and 250kDa.

In a preferred embodiment, the activated serotype 15B capsular polysaccharide comprises at least 0.6 mM acetate per mM of said serotype 15B capsular polysaccharide. In a preferred embodiment, the activated serotype 15B capsular polysaccharide comprises at least 0.7 mM acetate per mM of said serotype 15B capsular polysaccharide. In a preferred embodiment, the presence of O-acetyl groups is determined by ion-HPLC analysis.

In a preferred embodiment, the activated serotype 15B capsular polysaccharide comprises at least 0.1, 0.2, 0.3, 0.4, 0.5, 0.6, 0.7 or 0.8 mM glycerol per mM of said serotype 15B capsular polysaccharide. In a preferred embodiment, the activated serotype 15B capsular polysaccharide comprises at least 0.5, 0.6 or 0.7 mM glycerol per mM of said serotype 15B capsular polysaccharide. In a preferred embodiment, the activated serotype 15B capsular polysaccharide comprises at least 0.6 mM glycerol per mM of said serotype 15B capsular polysaccharide. In a preferred embodiment, the activated serotype 15B capsular polysaccharide comprises at least 0.7 mM glycerol per mM of said serotype 15B capsular polysaccharide.

In a preferred embodiment, the activated serotype 15B capsular polysaccharide has a molecular weight between 100 and 250 kDa and comprises at least 0.6 mM acetate per mM of said serotype 15B capsular polysaccharide.

In a preferred embodiment, the activated serotype 15B capsular polysaccharide has a molecular weight between 100 and 250 kDa and comprises at least 0.6 mM glycerol per mM of said serotype 15B capsular polysaccharide.

In a preferred embodiment, the activated serotype 15B capsular polysaccharide comprises at least 0.6 mM acetate per mM of said serotype 15B capsular polysaccharide and at least 0.6 mM glycerol per mM of said serotype 15B capsular polysaccharide.

In a preferred embodiment, the activated serotype 15B capsular polysaccharide has a molecular weight between 100 and 250 kDa and comprises at least 0.6 mM acetate per mM of said serotype 15B capsular polysaccharide and at least 0.6 mM glycerol per mM of said serotype 15B capsular polysaccharide.

In an embodiment, the activated serotype 15B capsular polysaccharide is lyophilized, optionally in the presence of cryoprotectant/lyoprotectant. In an embodiment, said cryoprotectant/lyoprotectant is a saccharide. In a preferred embodiment, the saccharide is selected from sucrose, trehalose, raffinose, stachyose, melezitose, dextran, mannitol, lactitol and palatinit. In a preferred embodiment, the saccharide is sucrose. The lyophilized activated capsular polysaccharide can then be compounded with a solution comprising the carrier protein.

In another embodiment, the activated serotype 15B capsular polysaccharide is compounded with the carrier protein and lyophilized optionally in the presence of cryoprotectant/lyoprotectant. In an embodiment, said cryoprotectant/lyoprotectant is a saccharide. In a preferred embodiment, the saccharide is selected from sucrose, trehalose, raffinose, stachyose, melezitose, dextran, mannitol, lactitol and palatinit. In a preferred embodiment, the saccharide is sucrose. The co-lyophilized polysaccharide and carrier protein can then be resuspended in solution and reacted with a reducing agent.

In an embodiment, the disclosure relates to a lyophilized activated serotype 15B capsular polysaccharide.

In an embodiment the disclosure relates to the co-lyophilized activated serotype 15B capsular polysaccharide and protein carrier. In a preferred embodiment, the protein carrier is CRM₁₉₇.

### Conjugation of activated serotype 15B capsular polysaccharide with a carrier protein

The activated serotype 15B capsular polysaccharide can be conjugated to a carrier protein by a process comprising the step of:
(a) compounding the activated serotype 15B capsular polysaccharide with a carrier protein, and,
(b) reacting the compounded activated serotype 15B capsular polysaccharide and carrier protein with a reducing agent to form a serotype 15B capsular polysaccharide-carrier protein conjugate.

The conjugation of activated serotype 15B capsular polysaccharide with a protein carrier by reductive amination in dimethylsulfoxide (DMSO) is suitable to preserve the O-acetyl content of the polysaccharide as compared for example to reductive amination in aqueous solution where the level of O-acetylation of the polysaccharide is significantly reduced. step (a) and step (b) are carried out in DMSO.

In a preferred embodiment, step (a) comprises dissolving lyophilized serotype 15B capsular polysaccharide in a solution comprising a carrier protein and DMSO. In a preferred embodiment, step (a) comprises dissolving co-lyophilized serotype 15B capsular polysaccharide and carrier protein in DMSO.

In a preferred embodiment, the concentration of activated serotype 15B capsular polysaccharide in step (b) is between 0.1 and 10 mg/mL, 0.5 and 5 mg/mL, 0.5 and 2 mg/mL. In a preferred embodiment, the concentration of activated serotype 15B capsular polysaccharide in step (b) is about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 mg/mL.

In a preferred embodiment the initial input ratio (weight by weight) of activated serotype 15B capsular polysaccharide to carrier protein is between 5:1 and 0.1:1, 2:1 and 0.1:1, 2:1 and 1:1, 1.5:1 and 1:1, 0.1:1 and 1:1, 0.3:1 and 1:1, 0.6:1 and 1:1.

In a preferred embodiment the initial input ratio of activated serotype 15B capsular polysaccharide to carrier protein is about 0.6:1 to 1.5:1, preferably 0.6:1 to 1:1. Such initial input ratio is particularly suitable to obtain low levels of free polysaccharide in the immunogenic conjugate.

In a preferred embodiment the initial input ratio of activated serotype 15B capsular polysaccharide to carrier protein is about 0.4:1, 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1 or2:1.

In an embodiment, the reducing agent is sodium cyanoborohydride, sodium triacetoxyborohydride, sodium or zinc borohydride in the presence of Bronsted or Lewis acids, amine boranes such as pyridine borane, 2-Picoline Borane, 2,6-diborane-methanol, dimethylamine-borane, t-BuMeⁱPrN-BH₃, benzylamine-BH₃ or 5-ethyl-2-methylpyridine borane (PEMB). In a preferred embodiment, the reducing agent is sodium cyanoborohydride. In a preferred embodiment, the reducing agent is sodium 2-Picoline Borane.

In a preferred embodiment, the quantity of reducing agent used in step (b) is between about 0.1 and 10 molar equivalents, 0.5 and 5 molar equivalents, 1 and 2 molar equivalents. In a preferred embodiment, the quantity of reducing agent used in step (b) is about 1, 1.1, 1.2, 1.3, 1.4, 1.5, 1.6, 1.7, 1.8, 1.9 or 2 molar equivalents.

In a preferred embodiment, the duration of step (b) is between 1 and 60 hours, 10 and 50 hours, 40 and 50 hours; 42 and 46 hours. In a preferred embodiment, the duration of step (b) is about 44 hours.

In a preferred embodiment, the temperature of the reaction in step (b) is maintained between 10 and 40°C, 15 and 30°C or 20 and 26°C. In a preferred embodiment, the temperature of the reaction in step (b) is maintained at about 23°C.

In a preferred embodiment, the process for the preparation of an immunogenic conjugate comprising *Streptococcus pneumoniae* serotype 15B capsular polysaccharide covalently linked to a carrier protein further comprises a step (step (c)) of capping unreacted aldehyde (quenching) by addition of NaBH₄.

In a preferred embodiment, the quantity of NaBH₄ used in step (c) is between 0.1 and 10 molar equivalents, 0.5 and 5 molar equivalent 1 and 3 molar equivalents. In a preferred embodiment, the quantity of NaBH₄ used in step (c) is about 2 molar equivalents.

In a preferred embodiment, the duration of step (c) is between 0.1 and 10 hours, 0.5 and 5 hours, 2 and 4 hours. In a preferred embodiment, the duration of step (c) is about 3 hours.

In a preferred embodiment, the temperature of the reaction in step (c) is maintained between 15 and 45°C, 15 and 30°C or 20 and 26°C. In a preferred embodiment, the temperature of the reaction in step (c) is maintained at about 23°C.

In a preferred embodiment the yield of the conjugation step (step b) is greater than 50%, 55%, 60%, 65%, 70%, 75%, 80%, 85% or 90%. In a preferred embodiment the yield of the conjugation step (step b) is greater than 60%. In a preferred embodiment the yield of the conjugation step (step b) is greater than 70%. The yield is the amount of serotype 15B polysaccharide in the conjugate x100 / amount of activated polysaccharide used in the conjugation step.

In a preferred embodiment, the process for the preparation of an immunogenic conjugate comprising *Streptococcus pneumoniae* serotype 15B capsular polysaccharide covalently linked to a carrier protein comprises the steps of:
(a) preparing a fermentation culture of serotype 15B *Streptococcus pneumonia* bacterial cells;
(b) lysing the bacterial cells in said fermentation culture;
(c) purifying serotype 15B polysaccharide from the fermentation culture;
(d) sizing the purified serotype 15B polysaccharide by high pressure homogenization;
(e) reacting the sized serotype 15B polysaccharide with an oxidizing agent;
(f) compounding the activated serotype 15B polysaccharide with a carrier protein, and,
(g) reacting the compounded activated serotype 15B polysaccharide and carrier protein with a reducing agent to form a serotype 15B polysaccharide-carrier protein conjugate; and,
(h) capping unreacted aldehyde (quenching) by addition of NaBH₄.

In a preferred embodiment the yield of the conjugation step (step g) of the above process is greater than 50%, 55%, 60%, 65%, 70%, 75%, 80%, 85% or 90%. In a preferred embodiment the yield of the conjugation step (step g) is greater than 60%. In a preferred embodiment the yield of the conjugation step (step g) is greater than 70%. The yield is the amount of serotype 15B polysaccharide in the conjugate x100) / amount of activated polysaccharide used in the conjugation step.

After conjugation of the serotype 15B capsular polysaccharide to the carrier protein, the polysaccharide-protein conjugate can be purified (enriched with respect to the amount of polysaccharide-protein conjugate) by a variety of techniques known to the skilled person. These techniques include dialysis, concentration/diafiltration operations, tangential flow filtration, precipitation/elution, column chromatography (DEAE or hydrophobic interaction chromatography), and depth filtration.

In a preferred embodiment the carrier protein is non-toxic and non-reactogenic and obtainable in sufficient amount and purity. Carrier proteins should be amenable to standard conjugation procedures.

In a preferred embodiment, the activated serotype 15B capsular polysaccharide is conjugated to a carrier protein which is selected in the group consisiting of: DT (Diphtheria toxin), TT (tetanus toxid) or fragment C of TT, CRM₁₉₇ (a nontoxic but antigenically identical variant of diphtheria toxin) other DT point mutants, such as CRM176, CRM228, CRM 45 (Uchida et al J. Biol. Chem. 218; 3838-3844, 1973); CRM 9, CRM102, CRM 103 and CRM107 and other mutations described by Nicholls and Youle in Genetically Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711, pneumococcal pneumolysin (Kuo et al (1995) Infect Immun 63; 2706-13) including ply detoxified in some fashion for example dPLY-GMBS (WO 04081515, PCT/EP2005/010258) or dPLY-formol, PhtX, including PhtA, PhtB, PhtD, PhtE (sequences of PhtA, PhtB, PhtD or PhtE are disclosed in WO 00/37105 or WO 00/39299) and fusions of Pht proteins for example PhtDE fusions, PhtBE fusions, Pht A-E (WO 01/98334, WO 03/54007, WO2009/000826), OMPC (meningococcal outer membrane protein - usually extracted from N.meningitidis serogroup B - EP0372501 ), PorB (from N. meningitidis), PD (Haemophilus influenza protein D - see, e.g., EP 0 594 610 B), or immunologically functional equivalents thereof, synthetic peptides (EP0378881 , EP0427347), heat shock proteins (WO 93/17712, WO 94/03208), pertussis proteins (WO 98/58668, EP0471 177), cytokines, lymphokines, growth factors or hormones (WO10 91/01146), artificial proteins comprising multiple human CD4+ T cell epitopes from various pathogen derived antigens (Falugi et al (2001) Eur J Immunol 31 ; 3816-3824) such as N19 protein (Baraldoi et al (2004) Infect Immun 72; 4884-7) pneumococcal surface protein PspA (WO 02/091998), iron uptake proteins (WO 01/72337), toxin A or B of C. difficile (WO 00/61761). In an embodiment, the activated serotype 15B capsular polysaccharide is conjugated to DT (Diphtheria toxoid). In another embodiment, the activated serotype 15B capsular polysaccharide is conjugated to TT (tetanus toxid). In another embodiment, the activated serotype 15B capsular polysaccharide is conjugated to fragment C of TT. In another embodiment, the activated serotype 15B capsular polysaccharide is conjugated to PD (Haemophilus influenza protein D - see, e.g., EP 0 594 610 B).

In a preferred embodiment, the activated serotype 15B capsular polysaccharide of the disclosure is conjugated to CRM₁₉₇ protein. The CRM₁₉₇ protein is a nontoxic form of diphtheria toxin but is immunologically indistinguishable from the diphtheria toxin. CRM₁₉₇ is produced by C. diphtheriae infected by the nontoxigenic phage β197^{tox-} created by nitrosoguanidine mutagenesis of the toxigenic corynephage beta (Uchida, T. et al. 1971, Nature New Biology 233:8-11). CRM₁₉₇ is purified through ultrafiltration, ammonium sulfate precipitation, and ion-exchange chromatography. The CRM₁₉₇ protein has the same molecular weight as the diphtheria toxin but differs therefrom by a single base change (guanine to adenine) in the structural gene. This single base change causes an amino acid substitution glutamic acid for glycine) in the mature protein and eliminates the toxic properties of diphtheria toxin. The CRM₁₉₇ protein is a safe and effective T-cell dependent carrier for saccharides. Further details about CRM₁₉₇ and production thereof can be found e.g. in US 5,614,382.

In an embodiment, the invention relate to an immunogenic conjugate, obtained by the process of the invention, comprising *Streptococcus pneumoniae* serotype 15B capsular polysaccharide covalently linked to a carrier protein. In an embodiment, the invention relate to an immunogenic conjugate, obtained by the process of the invention, comprising *Streptococcus pneumoniae* serotype 15B capsular polysaccharide covalently linked to a carrier protein by reductive amination. In an embodiment, the invention relate to an immunogenic conjugate, obtained by the process of the invention, comprising *Streptococcus pneumoniae* serotype 15B capsular polysaccharide covalently linked to a carrier protein by reductive amination in DMSO. In a preferred embodiment, the carrier protein is CRM₁₉₇. In a preferred embodiment of the disclosure, the polysaccharide is an isolated serotype 15B capsular polysaccharide as defined herein. In a preferred embodiment, of the disclosure the polysaccharide is an isolated serotype 15B capsular polysaccharide as defined herein which has been sized by high pressure homogenization.

In a preferred embodiment, the immunogenic conjugate comprises less than about 50, 45, 40, 35, 30, 25, 20 or 15% of free serotype 15B capsular polysaccharide compared to the total amount of serotype 15B capsular polysaccharide. In a preferred embodiment the immunogenic conjugate comprises less than about 25% of free serotype 15B capsular polysaccharide compared to the total amount of serotype 15B capsular polysaccharide. In a preferred embodiment the immunogenic conjugate comprises less than about 20% of free serotype 15B capsular polysaccharide compared to the total amount of serotype 15B capsular polysaccharide. In a preferred embodiment the immunogenic conjugate comprises less than about 15% of free serotype 15B capsular polysaccharide compared to the total amount of serotype 15B capsular polysaccharide.

In a preferred embodiment, the immunogenic conjugate has a molecular weight between 3000 and 20000kDa; 5000 and 10000kDa; 5000 and 20000kDa; 8000 and 20000kDa; 8000 and 16000 KDa; or 10000 and 16000 KDa. The molecular weight of the immunogenic conjugate is measured by SEC-MALLS.

In a preferred embodiment, the ratio (weight by weight) of serotype 15B capsular polysaccharide to carrier protein in the conjugate is between 0.5 and 3. In a preferred embodiment, the ratio of serotype 15B capsular polysaccharide to carrier protein in the conjugate is between 0.4 and 2, 0.5 and 2, 0.5 and 1.5, 0.5 and 1, 1 and 1.5, 1 and 2. In a preferred embodiment, the ratio of serotype 15B capsular polysaccharide to carrier protein in the conjugate is between 0.7 and 0.9.

Size exclusion chromatography media (CL-4B) can be used to determine the relative molecular size distribution of the conjugate. Size Exclusion Chromatography (SEC) is used in gravity fed columns to profile the molecular size distribution of conjugates. Large molecules excluded from the pores in the media elute more quickly than small molecules. Fraction collectors are used to collect the column eluate. The fractions are tested colorimetrically by saccharide assay. For the determination of Kd, columns are calibrated to establish the fraction at which molecules are fully excluded (V₀), (Kd=0), and the fraction representing the maximum retention (Vᵢ), (Kd=1). The fraction at which a specified sample attribute is reached (Vₑ), is related to Kd by the expression, Kd = (Vₑ - V₀)/ (Vᵢ - V₀).

In a preferred embodiment, at least 20% of the immunogenic conjugate has a Kd below or equal to 0.3 in a CL-4B column. In a preferred embodiment, at least 30% of the immunogenic conjugate has a Kd below or equal to 0.3 in a CL-4B column. In a preferred embodiment, at least 40% of the immunogenic conjugate has a Kd below or equal to 0.3 in a CL-4B column. In a preferred embodiment, at least 40%, 45%, 50%, 55%, 60%, 65%, 70%, 75%, 80%, or 85% of the immunogenic conjugate has a Kd below or equal to 0.3 in a CL-4B column. In a preferred embodiment, at least 60% of the immunogenic conjugate has a Kd below or equal to 0.3 in a CL-4B column. In a preferred embodiment, at least 70% of the immunogenic conjugate has a Kd below or equal to 0.3 in a CL-4B column.

In a preferred embodiment, between 40% and 90% of the serotype 15B immunogenic conjugate has a Kd below or equal to 0.3 in a CL-4B column. In a preferred embodiment, between 50% and 90% of the serotype 15B immunogenic conjugate has a Kd below or equal to 0.3 in a CL-4B column. In a preferred embodiment, between 65% and 80% of the serotype 15B immunogenic conjugate has a Kd below or equal to 0.3 in a CL-4B column.

The immunogenic conjugate comprises at least 0.6 mM acetate per mM serotype 15B capsular polysaccharide. In a preferred embodiment, the immunogenic conjugate comprises at least 0.7 mM acetate per mM serotype 15B capsular polysaccharide. In a preferred embodiment, the presence of O-acetyl groups is determined by ion-HPLC analysis.

In a preferred embodiment, the ratio of mM acetate per mM serotype 15B capsular polysaccharide in the immunogenic conjugate to mM acetate per mM serotype 15B capsular polysaccharide in the isolated polysaccharide is at least 0.6, 0.65, 0.7, 0.75, 0.8, 0.85, 0.9, or 0.95. In a preferred embodiment, the ratio of mM acetate per mM serotype 15B capsular polysaccharide in the immunogenic conjugate to mM acetate per mM serotype 15B capsular polysaccharide in the isolated polysaccharide is at least 0.7. In a preferred embodiment, the ratio of mM acetate per mM serotype 15B capsular polysaccharide in the immunogenic conjugate to mM acetate per mM serotype 15B capsular polysaccharide in the isolated polysaccharide is at least 0.9. In a preferred embodiment, the presence of O-acetyl groups is determined by ion-HPLC analysis.

In a preferred embodiment, the ratio of mM acetate per mM serotype 15B capsular polysaccharide in the immunogenic conjugate to mM acetate per mM serotype 15B capsular polysaccharide in the activated polysaccharide is at least 0.6, 0.65, 0.7, 0.75, 0.8, 0.85, 0.9, or 0.95. In a preferred embodiment, the ratio of mM acetate per mM serotype 15B capsular polysaccharide in the immunogenic conjugate to mM acetate per mM serotype 15B capsular polysaccharide in the activated polysaccharide is at least 0.7. In a preferred embodiment, the ratio of mM acetate per mM serotype 15B capsular polysaccharide in the immunogenic conjugate to mM acetate per mM serotype 15B capsular polysaccharide in the activated polysaccharide is at least 0.9. In a preferred embodiment, the presence of O-acetyl groups is determined by ion-HPLC analysis.

In a preferred embodiment, the immunogenic conjugate comprises at least 0.1, 0.2, 0.3, 0.4, 0.5, 0.6, 0.7 or 0.8 mM glycerol per mM serotype 15B capsular polysaccharide. In a preferred embodiment, the immunogenic conjugate comprises at least 0.5, 0.6 or 0.7 mM glycerol per mM serotype 15B capsular polysaccharide. In a preferred embodiment, the immunogenic conjugate comprises at least 0.6 mM glycerol per mM serotype 15B capsular polysaccharide. In a preferred embodiment, the immunogenic conjugate comprises at least 0.7 mM glycerol per mM serotype 15B capsular polysaccharide.

The degree of conjugation is the number of lysine residues in the carrier protein that are conjugated to serotype 15B capsular polysaccharide. The evidence for lysine modification of the carrier protein, due to covalent linkages to the polysaccharides, is obtained by amino acid analysis using routine methods known to those of skill in the art. Conjugation results in a reduction in the number of lysine residues recovered, compared to the CRM₁₉₇ protein starting material used to generate the conjugate materials.

In a preferred embodiment, the degree of conjugation of the immunogenic conjugate is between 2 and 15, 2 and 13, 2 and 10, 2 and 8, 2 and 6, 2 and 5, 2 and 4, 3 and 15, 3 and 13, 3 and 10, 3 and 8, 3 and 6, 3 and 5, 3 and 4, 5 and 15, 5 an 10, 8 and 15, 8 and 12, 10 and 15 or 10 and 12. In a preferred embodiment, the degree of conjugation of the immunogenic conjugate is between 2 and 5.

### Immunogenic composition

The term "immunogenic composition" relates to any pharmaceutical composition containing an antigen, e.g., a microorganism or a component thereof, which composition can be used to elicit an immune response in a subject.

As used herein, "immunogenic" means an ability of an antigen (or an epitope of the antigen), such as a bacterial capsular polysaccharide, or an immunogenic conjugate or immunogenic composition comprising an antigen, to elicit an immune response in a host such as a mammal, either humorally or cellularly mediated, or both.

In an embodiment, the disclosure relate to an immunogenic composition comprising an immunogenic serotype 15B capsular polysaccharide-carrier protein conjugate obtained by the process of the invention.

In an embodiment, the immunogenic composition disclosed herein, when administered to a subject, induces the formation of antibodies capable of binding to serotype 15B *Streptococcus pneumonia.* In an embodiment, the immunogenic composition disclosed herein, when administered to a subject, induces the formation of antibodies capable of binding to serotype 15B *Streptococcus pneumonia* as measured by a standard ELISA assay.

In an embodiment, the immunogenic composition disclosed herein, when administered to a subject, induces the formation of antibodies capable of binding to serotype 15B and 15A and/or 15C *Streptococcus pneumonia.* In an embodiment, the immunogenic composition disclosed herein, when administered to a subject, induces the formation of antibodies capable of binding to serotype 15B and 15A and/or 15C *Streptococcus pneumonia* as measured by a standard ELISA assay.

In an embodiment, the immunogenic composition disclosed herein, when administered to a subject, induces the formation of antibodies capable of binding to serotype 15B and 15C *Streptococcus pneumonia.* In an embodiment, the immunogenic composition disclosed herein, when administered to a subject, induces the formation of antibodies capable of binding to serotype 15B and 15C *Streptococcus pneumonia* as measured by a standard ELISA assay.

In the ELISA (Enzyme-linked Immunosorbent Assay) method, antibodies from the sera of vaccinated subjects are incubated with polysaccharides which have been adsorbed to a solid support. The bound antibodies are detected using enzyme-conjugated secondary detection antibodies.

In an embodiment said ELISA assay is the standardized (WHO) ELISA assay as defined by the WHO in the 'Training manual for Enzyme linked immunosorbent assay for the quantitation of Streptococcus pneumoniae serotype specific IgG (Pn PS ELISA).' (accessible at http://www.vaccine.uab.edu/ELISA%20protocol.pdf; accessed on March 31st, 2014).

The ELISA measures type specific IgG anti-*S*. *pneumoniae* capsular polysaccharide (PS) antibodies present in human serum. When dilutions of human sera are added to type-specific capsular PS-coated microtiter plates, antibodies specific for that capsular PS bind to the microtiter plates. The antibodies bound to the plates are detected using a goat anti-human IgG alkaline phosphatase-labeled antibody followed by a p-nitrophenyl phosphate substrate. The optical density of the colored end product is proportional to the amount of anticapsular PS antibody present in the serum.

In an embodiment, the immunogenic composition of the invention is able to elicit IgG antibodies in human which are capable of binding *S. pneumoniae* serotypes 15B polysaccharide at a concentration of at least 0.05, 0.1, 0.2, 0.3, 0.35, 0.4 or 0.5 µg/ml as determined by ELISA assay.

In an embodiment, the immunogenic composition of the invention is able to elicit IgG antibodies in human which are capable of binding *S. pneumoniae* serotypes 15C polysaccharide at a concentration of at least 0.05, 0.1, 0.2, 0.3, 0.35, 0.4 or 0.5 µg/ml as determined by ELISA assay.

In an embodiment, the immunogenic composition of the invention is able to elicit IgG antibodies in human which are capable of binding *S. pneumoniae* serotypes 15B and 15C polysaccharide at a concentration of at least 0.05, 0.1, 0.2, 0.3, 0.35, 0.4 or 0.5 µg/ml as determined by ELISA assay.

In an embodiment, the immunogenic composition disclosed herein, when administered to a subject, induces the formation of antibodies capable of killing serotype 15B *Streptococcus* pneumonia in an opsonophagocytosis assay (OPA) as disclosed herein. In an embodiment, the immunogenic composition disclosed herein, when tested in an OPA assay as disclosed herein, has an OPA titer greater than the OPA titer obtained with an unconjugated native *Streptococcus pneumonia* serotype 15B capsular polysaccharide.

In an embodiment, the immunogenic composition disclosed herein, when administered to a subject, induces the formation of antibodies capable of killing serotype 15C *Streptococcus* pneumonia in an opsonophagocytosis assay as disclosed herein. In an embodiment, the immunogenic composition disclosed herein, when tested in an OPA assay as disclosed herein, has an OPA titer greater than the OPA titer obtained with an unconjugated native *Streptococcus pneumonia* serotype 15C capsular polysaccharide.

In an embodiment, the immunogenic composition disclosed herein, when administered to a subject, induces the formation of antibodies capable of killing serotype 15B and 15C and/or 15A *Streptococcus pneumonia* in an opsonophagocytosis assay as disclosed herein.

In an embodiment, the immunogenic composition disclosed herein, when administered to a subject, induces the formation of antibodies capable of killing serotype 15B and 15C.

The pneumococcal opsonophagocytic assay (OPA), which measures killing of S. *pneumoniae* cells by phagocytic effector cells in the presence of functional antibody and complement, is considered to be an important surrogate for evaluating the effectiveness of pneumococcal vaccines.

Opsonophagocytic assay (OPA) can be conducted by incubating together a mixture of *Streptococcus pneumoniae* cells, a heat inactivated human serum to be tested, differentiated HL-60 cells (phagocytes) and an exogenous complement source (e.g. baby rabbit complement). Opsonophagocytosis proceeds during incubation and bacterial cells that are coated with antibody and complement are killed upon opsonophagocytosis. Colony forming units (cfu) of surviving bacteria that escape from opsonophagocytosis are determined by plating the assay mixture. The OPA titer is defined as the reciprocal dilution that results in a 50% reduction in bacterial count over control wells without test serum. The OPA titer is interpolated from the two dilutions that encompass this 50% killing cut-off.

An endpoint titer of 1:8 or greater is considered a positive result in these killing type OPA.

In an embodiment, the immunogenic composition of the invention is able to elicit a titer of at least 1:8 against *S. pneumoniae* serotype 15B in at least 50% of the subjects as determined by opsonophagocytic killing assay (OPA). In an embodiment, the immunogenic composition of the invention is able to elicit a titer of at least 1:8 against *S. pneumoniae* serotype 15B in at least 60%; 70%, 80%, 90%, or at least 93% of the subjects as determined by opsonophagocytic killing assay (OPA).

In an embodiment, the immunogenic composition of the invention is able to elicit a titer of at least 1:8 against *S. pneumoniae* serotype 15C in at least 50% of the subjects as determined by opsonophagocytic killing assay (OPA). In an embodiment, the immunogenic composition of the invention is able to elicit a titer of at least 1:8 against *S. pneumoniae* serotype 15C in at least 60%; 70%, 80%, 90%, or at least 95% of the subjects as determined by opsonophagocytic killing assay (OPA).

Formulation of the immunogenic composition of the present invention can be accomplished using art-recognized methods. For instance, the immunogenic conjugates of the invention can be formulated with a physiologically acceptable vehicle to prepare the composition. Examples of such vehicles include, but are not limited to, water, buffered saline, polyols (e.g., glycerol, propylene glycol, liquid polyethylene glycol) and dextrose solutions.

In a preferred embodiment, the immunogenic composition may comprise at least one additional antigen. In a preferred embodiments, the immunogenic composition may comprises at least one additional *Streptococcus pneumoniae* capsular polysaccharide.

In a preferred embodiment, the immunogenic composition may comprise at least one additional *Streptococcus pneumoniae* capsular polysaccharide conjugated to a carrier protein. In a preferred embodiment, said carrier protein is CRM₁₉₇.

In certain embodiments, the immunogenic composition comprises one or more adjuvants. As defined herein, an "adjuvant" is a substance that serves to enhance the immunogenicity of an immunogenic composition of this invention. Thus, adjuvants are often given to boost the immune response and are well known to the skilled artisan. Suitable adjuvants to enhance effectiveness of the composition include, but are not limited to:
(1) aluminum salts (alum), such as aluminum hydroxide, aluminum phosphate, aluminum sulfate, etc.;
(2) oil-in-water emulsion formulations (with or without other specific immunostimulating agents such as muramyl peptides (defined below) or bacterial cell wall components), such as, for example,
   (a) MF59 (PCT Pub. No. WO 90/14837), containing 5% Squalene, 0.5%5 Tween 80, and 0.5% Span 85 (optionally containing various amounts of MTP-PE (see below, although not required)) formulated into submicron particles using a microfluidizer such as Model 11OY microfluidizer (Microfluidics, Newton, MA),
   (b) SAF, containing 10% Squalene, 0.4% Tween 80, 5% pluronic-blocked polymer L121, and thr-MDP (see below) either microfluidized into a submicron emulsion or vortexed to generate a larger particle size emulsion, and
   (c) Ribi™ adjuvant system (RAS), (Corixa, Hamilton, MT) containing 2% Squalene, 0.2% Tween 80, and one or more bacterial cell wall components from the group consisting of 3-O-deaylated monophosphorylipid A (MPL™) described in U.S. Patent No. 4,912,094 (Corixa), trehalose dimycolate (TDM), and cell wall skeleton (CWS), preferably MPL + CWS (Detox™);
(3) saponin adjuvants, such as Quil A or STIMULON™ QS-21 (Antigenics, Framingham, MA) (U.S. Patent No. 5,057,540) may be used or particles generated therefrom such as ISCOMs (immunostimulating complexes);
(4) bacteriallipopolysaccharides, synthetic lipid A analogs such as aminoalkyl glucosamine phosphate compounds (AGP), or derivatives or analogs thereof, which are available from Corixa, and which are described in U.S. Patent No.6, 113,918; one such AGP is 2-[(R)-3-Tetradecanoyloxytetradecanoylamino]ethyl 2-Deoxy-4-Ophosphono-3-O-[(R)-3-tetradecanoyloxytetradecanoyl]-2-[(R)-3tetradecanoyloxytetradecanoylamino]-b-D-glucopyranoside, which is also know as 529 (formerly known as RC529), which is formulated as an aqueous form or as a stable emulsion, synthetic polynucleotides such as oligonucleotides containing CpG motif(s) (U.S. Patent No. 6,207,646);
(5) cytokines, such as interleukins (e.g., IL-1, IL-2, IL-4, IL-5, IL-6, IL-7, IL-12, IL-15, IL-18, etc.), interferons (e.g., gamma interferon), granulocyte macrophage colony stimulating factor (GM-CSF), macrophage colony stimulating factor (M-CSF), tumor necrosis factor (TNF), costimulatory molecules 87-1 and 87-2, etc.;
(6) detoxified mutants of a bacterial ADP-ribosylating toxin such as a cholera toxin (CT) either in a wild-type or mutant form, for example, where the glutamic acid at amino acid position 29 is replaced by another amino acid, preferably a histidine, in accordance with published international patent application number WO 00/18434 (see also WO 02/098368 and WO 02/098369), a pertussis toxin (PT), or an E. coli heat-labile toxin (LT), particularly LT-K63, LT-R72, CT-S109, PT-K9/G129 (see, e.g.,WO 93/13302 and WO 92/19265); and
(7) other substances that act as immunostimulating agents to enhance the effectiveness of the composition.

Muramyl peptides include, but are not limited to, N-acetyl-muramyl-L-threonyl-D-isoglutamine (thr-MDP), N-acetyl-normuramyl-L-alanine-2-(1'-2'-dipalmitoyl-*sn*-glycero-3-hydroxyphosphoryloxy)-ethylamine (MTP-PE), etc.

In an embodiment of the present invention, the immunogenic compositions as disclosed herein comprise a CpG Oligonucleotide as adjuvant. A CpG oligonucleotide as used herein refers to an immunostimulatory CpG oligodeoxynucleotide (CpG ODN), and accordingly these terms are used interchangeably unless otherwise indicated. Immunostimulatory CpG oligodeoxynucleotides contain one or more immunostimulatory CpG motifs that are unmethylated cytosine-guanine dinucleotides, optionally within certain preferred base contexts. The methylation status of the CpG immunostimulatory motif generally refers to the cytosine residue in the dinucleotide. An immunostimulatory oligonucleotide containing at least one unmethylated CpG dinucleotide is an oligonucleotide which contains a 5' unmethylated cytosine linked by a phosphate bond to a 3' guanine, and which activates the immune system through binding to Toll-like receptor 9 (TLR-9). In another embodiment the immunostimulatory oligonucleotide may contain one or more methylated CpG dinucleotides, which will activate the immune system through TLR9 but not as strongly as if the CpG motif(s) was/were unmethylated. CpG immunostimulatory oligonucleotides may comprise one or more palindromes that in turn may encompass the CpG dinucleotide. CpG oligonucleotides have been described in a number of issued patents, published patent applications, and other publications, including U.S. Patent Nos. 6,194,388; 6,207,646; 6,214,806; 6,218,371; 6,239,116; and 6,339,068.

In an embodiment of the present invention, the immunogenic compositions as disclosed herein comprise any of the CpG Oligonucleotide described at pages 3 lines 22 to page 12 line 36 of WO2010/125480.

Different classes of CpG immunostimulatory oligonucleotides have been identified. These are referred to as A, B, C and P class, and are described in greater detail at pages 3 lines 22 to page 12 line 36 of WO2010/125480. Methods of the invention embrace the use of these different classes of CpG immunostimulatory oligonucleotides.

In an embodiment of the present invention, the immunogenic compositions as disclosed herein comprise an A class CpG Oligonucleotide. Preferably, the "A class" CpG oligonucleotide of the invention has the following nucleic acid sequence: 5' GGGGACGACGTCGTGGGGGGG 3' (SEQ ID NO: 1). Some non-limiting examples of A-Class oligonucleotides include: 5' G*G*G_G_A_C_G_A_C_G_T_C_G_T_G_G*G*G*G*G*G 3' (SEQ ID NO: 2) ; wherein * refers to a phosphorothioate bond and _ refers to a phosphodiester bond.

In an embodiment of the present invention, the immunogenic compositions as disclosed herein comprise a B class CpG Oligonucleotide. In one embodiment, the CpG oligonucleotide for use in the present invention is a B class CpG oligonucleotide represented by at least the formula:
5' X₁X₂CGX₃X₄ 3', wherein X1, X2, X3, and X4 are nucleotides. In one embodiment, X₂ is adenine, guanine, or thymine. In another embodiment, X₃ is cytosine, adenine, or thymine. The B class CpG oligonucleotide sequences of the invention are those broadly described above in US patents Ps 6,194,388, 6,207,646, 6,214,806, 6,218,371, 6,239,116 and 6,339,068. Exemplary sequences include but are not limited to those disclosed in these latter applications and patents.

In an embodiment, the "B class" CpG oligonucleotide of the invention has the following nucleic acid sequence:
5' TCGTCGTTTTTCGGTGCTTTT 3' (SEQ ID NO: 3), or
5' TCGTCGTTTTTCGGTCGTTTT 3' (SEQ ID NO: 4), or
5' TCGTCGTTTTGTCGTTTTGTCGTT 3' (SEQ ID NO: 5), or
5' TCGTCGTTTCGTCGTTTTGTCGTT 3' (SEQ ID NO: 6), or
5' TCGTCGTTTTGTCGTTTTTTTCGA 3' (SEQ ID NO: 7).

In any of these sequences, all of the linkages may be all phosphorothioate bonds. In another embodiment, in any of these sequences, one or more of the linkages may be phosphodiester, preferably between the "C" and the "G" of the CpG motif making a semi-soft CpG oligonucleotide. In any of these sequences, an ethyl-uridine or a halogen may substitute for the 5' T; examples of halogen substitutions include but are not limited to bromo-uridine or iodo-uridine substitutions.

Some non-limiting examples of B-Class oligonucleotides include:
5' T*C*G*T*C*G*T*T*T*T*T*C*G*G*T*G*C*T*T*T*T 3' (SEQ ID NO: 8), or
5' T*C*G*T*C*G*T*T*T*T*T*C*G*G*T*C*G*T*T*T*T 3' (SEQ ID NO: 9), or
5' T*C*G*T*C*G*T*T*T*T*G*T*C*G*T*T*T*T*G*T*C*G*T*T 3' (SEQ ID NO: 10), or
5' T*C*G*T*C*G*T*T*T*C*G*T*C*G*T*T*T*T*G*T*C*G*T*T 3' (SEQ ID NO: 11), or
5' T*C*G*T*C*G*T*T*T*T*G*T*C*G*T*T*T*T*T*T*T*C*G*A 3' (SEQ ID NO: 12).
wherein * refers to a phosphorothioate bond.

In an embodiment of the present invention, the immunogenic compositions as disclosed herein comprise a C class CpG Oligonucleotide. In an embodiment, the "C class" CpG oligonucleotides of the invention has the following nucleic acid sequence:
5' TCGCGTCGTTCGGCGCGCGCCG 3' (SEQ ID NO: 13), or
5' TCGTCGACGTTCGGCGCGCGCCG 3' (SEQ ID NO: 14), or
5' TCGGACGTTCGGCGCGCGCCG 3' (SEQ ID NO: 15), or
5' TCGGACGTTCGGCGCGCCG 3' (SEQ ID NO: 16), or
5' TCGCGTCGTTCGGCGCGCCG 3' (SEQ ID NO: 17), or
5' TCGACGTTCGGCGCGCGCCG 3' (SEQ ID NO: 18), or
5' TCGACGTTCGGCGCGCCG 3' (SEQ ID NO: 19), or
5' TCGCGTCGTTCGGCGCCG 3' (SEQ ID NO: 20), or
5' TCGCGACGTTCGGCGCGCGCCG 3' (SEQ ID NO: 21), or
5' TCGTCGTTTTCGGCGCGCGCCG 3' (SEQ ID NO: 22), or
5' TCGTCGTTTTCGGCGGCCGCCG 3' (SEQ ID NO: 23), or
5' TCGTCGTTTTACGGCGCCGTGCCG 3' (SEQ ID NO: 24), or
5' TCGTCGTTTTCGGCGCGCGCCGT 3' (SEQ ID NO: 25).

In any of these sequences, all of the linkages may be all phosphorothioate bonds. In another embodiment, in any of these sequences, one or more of the linkages may be phosphodiester, preferably between the "C" and the "G" of the CpG motif making a semi-soft CpG oligonucleotide.

Some non-limiting examples of C-Class oligonucleotides include:
5' T*C_G*C_G*T*C_G*T*T*C_G*G*C*G*C_G*C*G*C*C*G 3' (SEQ ID NO: 26), or
5' T*C_G*T*C_G*A*C_G*T*T*C_G*G*C*G*C_G*C*G*C*C*G 3' (SEQ ID NO: 27), or
5' T*C_G*G*A*C_G*T*T*C_G*G*C*G*C_G*C*G*C*C*G 3' (SEQ ID NO: 28), or
5' T*C_G*G*A*C_G*T*T*C_G*G*C*G*C*G*C*C*G 3' (SEQ ID NO: 29), or
5' T*C_G*C_G*T*C_G*T*T*C_G*G*C*G*C*G*C*C*G 3' (SEQ ID NO: 30), or
5' T*C_G*A*C_G*T*T*C_G*G*C*G*C_G*C*G*C*C*G 3' (SEQ ID NO: 31), or
5' T*C_G*A*C_G*T*T*C_G*G*C*G*C*G*C*C*G 3' (SEQ ID NO: 32), or
5' T*C_G*C_G*T*C_G*T*T*C_G*G*C*G*C*C*G 3' (SEQ ID NO: 33), or
5' T*C_G*C_G*A*C_G*T*T*C_G*G*C*G*C_G*C*G*C*C*G 3' (SEQ ID NO: 34), or
5' T*C*G*T*C*G*T*T*T*T*C*G*G*C*G*C*G*C*G*C*C*G 3' (SEQ ID NO: 35), or
5' T*C*G*T*C*G*T*T*T*T*C*G*G*C*G*G*C*C*G*C*C*G 3' (SEQ ID NO: 36), or
5' T*C*G*T*C_G*T*T*T*T*A*C_G*G*C*G*C*C_G*T*G*C*C*G 3' (SEQ ID NO: 37), or
5' T*C_G*T*C*G*T*T*T*T*C*G*G*C*G*C*G*C*G*C*C*G*T 3' (SEQ ID NO: 38)
wherein * refers to a phosphorothioate bond and _ refers to a phosphodiester bond.

In any of these sequences, an ethyl-uridine or a halogen may substitute for the 5' T; examples of halogen substitutions include but are not limited to bromo-uridine or iodo-uridine substitutions.

In an embodiment of the present invention, the immunogenic compositions as disclosed herein comprise a P class CpG Oligonucleotide. In an embodiment, the CpG oligonucleotide for use in the present invention is a P class CpG oligonucleotide containing a 5' TLR activation domain and at least two palindromic regions, one palindromic region being a 5' palindromic region of at least 6 nucleotides in length and connected to a 3' palindromic region of at least 8 nucleotides in length either directly or through a spacer, wherein the oligonucleotide includes at least one YpR dinucleotide. In an embodiment, said oligoonucleotide is not T*C_G*T*C_G*A*C_G*T*T*C_G*G*C*G*C_G*C*G*C*C*G (SEQ ID NO: 27). In one embodiment the a P class CpG oligonucleotide includes at least one unmethylated CpG dinucleotide. In another embodiment the TLR activation domain is TCG, TTCG, TTTCG, TYpR, TTYpR, TTTYpR, UCG, UUCG, UUUCG, TTT, or TTTT. In yet another embodiment the TLR activation domain is within the 5' palindromic region. In another embodiment the TLR activation domain is immediately 5' to the 5' palindromic region.

In an embodiment, the "P class" CpG oligonucleotides of the invention has the following nucleic acid sequence: 5' TCGTCGACGATCGGCGCGCGCCG 3' (SEQ ID NO: 39).

In said sequences, all of the linkages may be all phosphorothioate bonds. In another embodiment, one or more of the linkages may be phosphodiester, preferably between the "C" and the "G" of the CpG motif making a semi-soft CpG oligonucleotide. In any of these sequences, an ethyl-uridine or a halogen may substitute for the 5' T; examples of halogen substitutions include but are not limited to bromo-uridine or iodo-uridine substitutions.

A non-limiting example of P-Class oligonucleotides include:
5' T*C_G*T*C_G*A*C_G*A*T*C_G*G*C*G*C_G*C*G*C*C*G 3' (SEQ ID NO: 40)
wherein * refers to a phosphorothioate bond and _ refers to a phosphodiester bond.

In one embodiment the oligonucleotide includes at least one phosphorothioate linkage. In another embodiment all internucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In one embodiment the lipophilic group is cholesterol.

In an embodiment, all the internucleotide linkage of the CpG oligonucleotides disclosed herein are phosphodiester bonds ("soft" oligonucleotides, as described in the PCT application WO2007/026190). In another embodiment, CpG oligonucleotides of the invention are rendered resistant to degradation (e.g., are stabilized). A "stabilized oligonucleotide " refers to an oligonucleotide that is relatively resistant to *in vivo* degradation (e.g. via an exo- or endo-nuclease). Nucleic acid stabilization can be accomplished via backbone modifications. Oligonucleotides having phosphorothioate linkages provide maximal activity and protect the oligonucleotide from degradation by intracellular exo- and endo-nucleases. The immunostimulatory oligonucleotides may have a chimeric backbone, which have combinations of phosphodiester and phosphorothioate linkages. For purposes of the instant invention, a chimeric backbone refers to a partially stabilized backbone, wherein at least one internucleotide linkage is phosphodiester or phosphodiester-like, and wherein at least one other internucleotide linkage is a stabilized internucleotide linkage, wherein the at least one phosphodiester or phosphodiester-like linkage and the at least one stabilized linkage are different. When the phosphodiester linkage is preferentially located within the CpG motif such molecules are called "semi-soft" as described in the PCT application WO2007/026190.

The size of the CpG oligonucleotide (i.e., the number of nucleotide residues along the length of the oligonucleotide) also may contribute to the stimulatory activity of the oligonucleotide. For facilitating uptake into cells, CpG oligonucleotide of the invention preferably have a minimum length of 6 nucleotide residues. Oligonucleotides of any size greater than 6 nucleotides (even many kb long) are capable of inducing an immune response if sufficient immunostimulatory motifs are present, because larger oligonucleotides are degraded inside cells. In certain embodiments, the CpG oligonucleotides are 6 to 100 nucleotides long, preferentially 8 to 30 nucleotides long. In important embodiments, nucleic acids and oligonucleotides of the invention are not plasmids or expression vectors.

In an embodiment, the CpG oligonucleotides disclosed herein comprise substitutions or modifications, such as in the bases and/or sugars as described at paragraph 134 to 147 of WO2007/026190.

In an embodiment, the CpG oligonucleotide is chemically modified. Examples of chemical modifications are known to the skilled person and are described, for example in Uhlmann E. et al. (1990), Chem. Rev. 90:543, S. Agrawal, Ed., Humana Press, Totowa, USA 1993; Crooke, S.T. et al. (1996) Annu. Rev. Pharmacol. Toxicol. 36:107-129; and Hunziker J. et al., (1995), Mod. Synth. Methods 7:331-417. An oligonucleotide according to the invention may have one or more modifications, wherein each modification is located at a particular phosphodiester internucleoside bridge and/or at a particular β-D-ribose unit and/or at a particular natural nucleoside base position in comparison to an oligonucleotide of the same sequence which is composed of natural DNA or RNA.

In some embodiments of the invention, CpG-containing nucleic acids might be simply mixed with immunogenic carriers according to methods known to those skilled in the art (see, e.g. WO03/024480).

In a particular embodiment of the present invention, any of the immunogenic composition disclosed herein comprises from 2µg to 100mg of CpG oligonucleotide, preferably from 0.1mg to 50 mg CpG oligonucleotide, preferably from 0.2mg to 10 mg CpG oligonucleotide, preferably from 0.3 mg to 5 mg CpG oligonucleotide, even preferably from 0.5 to 2 mg CpG oligonucleotide, even preferably from 0.75 to 1.5 mg CpG oligonucleotide. In a preferred embodiment, the immunogenic composition disclosed herein comprises approximately 1mg CpG oligonucleotide.

In a preferred embodiment, the adjuvant is an aluminum-based adjuvant selected from the group consisting of aluminum phosphate, aluminum sulfate and aluminum hydroxide. In one embodiment, the immunogenic compositions described herein comprise the adjuvant aluminum phosphate.

In a preferred embodiments, the immunogenic compositions of the invention further comprise at least one of a buffer, a cryoprotectant, a salt, a divalent cation, a non-ionic detergent, an inhibitor of free radical oxidation, a diluent or a carrier.

The immunogenic composition optionally can comprise one or more physiologically acceptable buffers selected from, but not limited to Tris (trimethamine), phosphate, acetate, borate, citrate, glycine, histidine and succinate. In certain embodiments, the formulation is buffered to within a pH range of about 5.0 to about 7.0, preferably from about 5.5 to about 6.5.

The immunogenic composition optionally can comprise one or more non-ionic surfactants, including but not limited to polyoxyethylene sorbitan fatty acid esters, Polysorbate-80 (Tween 80), Polysorbate-60 (Tween 60), Polysorbate-40 (Tween 40) and Polysorbate-20 (Tween 20), polyoxyethylene alkyl ethers, including but not limited to Brij 58, Brij 35, as well as others such as Triton X-100; Triton X- 114, NP40, Span 85 and the Pluronic series of non-ionic surfactants (e. g. , Pluronic 121). In a preferred embodiment, the immunogenic composition comprises Polysorbate-80 or Polysorbate-40, preferably Polysorbate-80. In a preferred embodiment, the immunogenic composition comprises Polysorbate-80 at a concentration from about 0.001% to about 2% (with up to about 0.25% being preferred) or Polysorbate-40 at a concentration from about 0.001% to 1% (with up to about 0.5% being preferred).

The invention further relates to vaccines comprising the immunogenic composition of the invention.

### Methods for inducing an immune response and protecting against infection

The present disclosure also includes methods of use for immunogenic compositions described herein. For example, one embodiment of the disclosure provides a method of inducing an immune response against *Streptococcus pneumoniae,* comprising administering to a subject an immunogenic amount of any of the immunogenic compositions described herein.

One embodiment of the disclosure provides a method of protecting a subject against an infection with *Streptococcus pneumoniae,* or a method of preventing infection with *Streptococcus pneumoniae,* or a method of reducing the severity of or delaying the onset of at least one symptom associated with an infection caused by *Streptococcus pneumoniae,* the methods comprising administering to a subject an immunogenic amount of any of the immunogenic compositions described herein.

One embodiment of the disclosure provides a method of protecting a subject against an infection with serotype 15B *Streptococcus pneumoniae,* or a method of preventing infection with serotype 15B *Streptococcus pneumoniae,* or a method of reducing the severity of or delaying the onset of at least one symptom associated with an infection caused by serotype 15B *Streptococcus pneumoniae,* the methods comprising administering to a subject an immunogenic amount of any of the immunogenic compositions described herein.

One embodiment of the disclosure provides a method of protecting a subject against an infection with serotype 15C *Streptococcus pneumoniae,* or a method of preventing infection with serotype 15C *Streptococcus pneumoniae,* or a method of reducing the severity of or delaying the onset of at least one symptom associated with an infection caused by serotype 15C *Streptococcus pneumoniae,* the methods comprising administering to a subject an immunogenic amount of any of the immunogenic compositions described herein.

One embodiment of the disclosure provides a method of protecting a subject against an infection with serotype 15A *Streptococcus pneumoniae,* or a method of preventing infection with serotype 15A *Streptococcus pneumoniae,* or a method of reducing the severity of or delaying the onset of at least one symptom associated with an infection caused by serotype 15A *Streptococcus pneumoniae,* the methods comprising administering to a subject an immunogenic amount of any of the immunogenic compositions described herein.

One embodiment of the disclosure provides a method of treating or preventing a *Streptococcus pneumoniae* infection, disease or condition associated with serotype 15A, 15B and/or 15C (preferably 15B and/or 15C, more preferably 15B) *Streptococcus pneumoniae* in a subject, the method comprising the step of administering a therapeutically or prophylactically effective amount of an immunogenic composition described herein to the subject. Another embodiment provides a method of treating or preventing a *Streptococcus pneumoniae* infection, disease or condition associated with a serotype 15A, 15B and/or 15C (preferably 15B and/or 15C, more preferably 15B) *Streptococcus pneumoniae* in a subject, the method comprising generating a polyclonal or monoclonal antibody preparation from the immunogenic composition described herein, and using said antibody preparation to confer passive immunity to the subject.

In one embodiment, the disclosure relates to the use of the immunogenic conjugate or immunogenic composition disclosed herein for the manufacture of a medicament for protecting a subject against an infection with *Streptococcus pneumoniae,* and/or preventing infection with *Streptococcus pneumoniae,* and/or reducing the severity of or delaying the onset of at least one symptom associated with an infection caused by *Streptococcus pneumoniae,* and/or protecting a subject against an infection with serotype 15A, 15B and/or 15C (preferably 15B and/or 15C, more preferably 15B) *Streptococcus pneumoniae* and/or preventing infection with serotype 15A, 15B and/or 15C (preferably 15B and/or 15C, more preferably 15B) *Streptococcus pneumoniae,* and/or reducing the severity of or delaying the onset of at least one symptom associated with an infection caused by serotype 15A, 15B and/or 15C (preferably 15B and/or 15C, more preferably 15B) *Streptococcus pneumoniae.*

In one embodiment, the disclosure relates to the use of the immunogenic conjugate or immunogenic composition disclosed herein for protecting a subject against an infection with *Streptococcus pneumoniae,* and/or preventing infection with *Streptococcus pneumoniae,* and/or reducing the severity of or delaying the onset of at least one symptom associated with an infection caused by *Streptococcus pneumoniae,* and/or protecting a subject against an infection with serotype 15A, 15B and/or 15C (preferably 15B and/or 15C, more preferably 15B) *Streptococcus pneumoniae* and/or preventing infection with serotype 15A, 15B and/or 15C (preferably 15B and/or 15C, more preferably 15B) *Streptococcus pneumoniae,* and/or reducing the severity of or delaying the onset of at least one symptom associated with an infection caused by serotype 15A, 15B and/or 15C (preferably 15B and/or 15C, more preferably 15B) *Streptococcus pneumoniae.*

An "immune response" to an immunogenic composition is the development in a subject of a humoral and/or a cell-mediated immune response to molecules present in the immunogenic composition or vaccine composition of interest. For purposes of the present disclosure, a "humoral immune response" is an antibody-mediated immune response and involves the induction and generation of antibodies that recognize and bind with some affinity for the antigen in the immunogenic composition or vaccine of the disclosure, while a "cell-mediated immune response" is one mediated by T-cells and/or other white blood cells. A "cell-mediated immune response" is elicited by the presentation of antigenic epitopes in association with Class I or Class II molecules of the major histocompatibility complex (MHC), CD1 or other non-classical MHC-like molecules. This activates antigen-specific CD4+ T helper cells or CD8+ cytotoxic T lymphocyte cells ("CTLs"). CTLs have specificity for peptide antigens that are presented in association with proteins encoded by classical or non-classical MHCs and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide or other antigens in association with classical or non-classical MHC molecules on their surface. A "cell-mediated immune response" also refers to the production of cytokines, chemokines and other such molecules produced by activated T-cells and/or other white blood cells, including those derived from CD4+ and CD8+ T-cells. The ability of a particular antigen or composition to stimulate a cell-mediated immunological response may be determined by a number of assays, such as by lymphoproliferation (lymphocyte activation) assays, CTL cytotoxic cell assays, by assaying for T-lymphocytes specific for the antigen in a sensitized subject, or by measurement of cytokine production by T cells in response to re-stimulation with antigen. Such assays are well known in the art. *See, e.g.,* Erickson et al. (1993) J. Immunol. 151:4189-4199; and Doe et al. (1994) Eur. J. Immunol. 24:2369-2376.

As used herein, "treatment" (including variations thereof, e.g., "treat" or "treated") means any one or more of the following: (i) the prevention of infection or re-infection, as in a traditional vaccine, (ii) the reduction in the severity of, or, in the elimination of symptoms, and (iii) the substantial or complete elimination of the pathogen or disorder in question. Hence, treatment may be effected prophylactically (prior to infection) or therapeutically (following infection). In the present disclosure, prophylactic treatment is the preferred mode. According to a particular embodiment of the present disclosure, compositions and methods are provided that treat, including prophylactically and/or therapeutically immunize, a host animal against a serotype 15A, 15B and/or 15C (preferably 15B and/or 15C, more preferably 15B) *Streptococcus pneumoniae* infection. The methods of the present disclosure are useful for conferring prophylactic and/or therapeutic immunity to a subject. The methods of the present disclosure can also be practiced on subjects for biomedical research applications.

An "immunogenic amount", and "immunologically effective amount," both of which are used interchangeably herein, refers to the amount of antigen or immunogenic composition sufficient to elicit an immune response, either a cellular (T-cell) or humoral (B-cell or antibody) response, or both, as measured by standard assays known to one skilled in the art.

In a preferred embodiment, said subject is a human. In a most preferred embodiment, said subject is a newborn (i.e. under three months of age), an infant (from 3 months to one year of age) or a toddler (i.e. from one year to four years of age).

In an embodiment, the immunogenic compositions disclosed herein are for use as a vaccine.

In such embodiment, the subject to be vaccinated may be less than 1 year of age. For example, the subject to be vaccinated can be about 1, 2, 3, 4, 5, 6, 7, 8, 9, 10, 11 or 12 months of age. In an embodiment, the subject to be vaccinated is about 2, 4 or 6 months of age. In another embodiment, the subject to be vaccinated is less than 2 years of age. For example the subject to be vaccinated can be about 12-15 months of age. In some cases, as little as one dose of the immunogenic composition according to the invention is needed, but under some circumstances, a second, third or fourth dose may be given (see regimen section).

In an embodiment of the present invention, the subject to be vaccinated is a human adult 50 years of age or older, more preferably a human adult 55 years of age or older. In an embodiment, the subject to be vaccinated is a human adult 65 years of age or older, 70 years of age or older, 75 years of age or older or 80 years of age or older.

In an embodiment the subject to be vaccinated is an immunocompromised individual, in particular a human. An immunocompromised individual is generally defined as a person who exhibits an attenuated or reduced ability to mount a normal humoral or cellular defense to challenge by infectious agents.

In an embodiment of the present invention, the immunocompromised subject to be vaccinated suffers from a disease or condition that impairs the immune system and results in an antibody response that is insufficient to protect against or treat pneumococcal disease.

In an embodiment, said disease is a primary immunodeficiency disorder. Preferably, said primary immunodeficiency disorder is selected from the group consisting of: combined T- and B-cell immunodeficiencies, antibody deficiencies, well-defined syndromes, immune dysregulation diseases, phagocyte disorders, innate immunity deficiencies, autoinflammatory disorders, and complement deficiencies. In an embodiment, said primary immunodeficiency disorder is selected from the one disclosed on page 24 line 11 to page 25 line 19 of the PCT application WO2010/125480.

In a particular embodiment of the present invention, the immunocompromised subject to be vaccinated suffers from a disease selected from the groups consisting of: HIV-infection, acquired immunodeficiency syndrome (AIDS), cancer, chronic heart or lung disorders, congestive heart failure, diabetes mellitus, chronic liver disease, alcoholism, cirrhosis, spinal fluid leaks, cardiomyopathy, chronic bronchitis, emphysema, Chronic obstructive pulmonary disease (COPD), spleen dysfunction (such as sickle cell disease), lack of spleen function (asplenia), blood malignancy, leukemia, multiple myeloma, Hodgkin's disease, lymphoma, kidney failure, nephrotic syndrome and asthma.

In an embodiment of the present invention, the immunocompromised subject to be vaccinated suffers from malnutrition.

In a particular embodiment of the present invention, the immunocompromised subject to be vaccinated is taking a drug or treatment that lowers the body's resistance to infection. In an embodiment, said drug is selected from the one disclosed on page 26 line 33 to page 26 line 40 of the PCT application WO2010/125480.

In a particular embodiment of the present invention, the immunocompromised subject to be vaccinated is a smoker.

In a particular embodiment of the present invention, the immunocompromised subject to be vaccinated has a white blood cell count (leukocyte count) below 5 x 10⁹ cells per liter, or below 4 x 10⁹ cells per liter, or below 3 x 10⁹ cells per liter, or below 2 x 10⁹ cells per liter, or below 1 x 10⁹ cells per liter, or below 0.5 x 10⁹ cells per liter, or below 0.3 x 10⁹ cells per liter, or below 0.1 x 10⁹ cells per liter.

White blood cell count (leukocyte count): The number of white blood cells (WBCs) in the blood. The WBC is usually measured as part of the CBC (complete blood count). White blood cells are the infection-fighting cells in the blood and are distinct from the red (oxygen-carrying) blood cells known as erythrocytes. There are different types of white blood cells, including neutrophils (polymorphonuclear leukocytes; PMNs), band cells (slightly immature neutrophils), T-type lymphocytes (T cells), B-type lymphocytes (B cells), monocytes, eosinophils, and basophils. All the types of white blood cells are reflected in the white blood cell count. The normal range for the white blood cell count is usually between 4,300 and 10,800 cells per cubic millimeter of blood. This can also be referred to as the leukocyte count and can be expressed in international units as 4.3 - 10.8 x 10⁹ cells per liter.

In a particular embodiment of the present invention, the immunocompromised subject to be vaccinated suffers from neutropenia. In a particular embodiment of the present invention, the immunocompromised subject to be vaccinated has a neutrophil count below 2 x 10⁹ cells per liter, or below 1 x 10⁹ cells per liter, or below 0.5 x 10⁹ cells per liter, or below 0.1 x 10⁹ cells per liter, or below 0.05 x 10⁹ cells per liter.

A low white blood cell count or "neutropenia" is a condition characterized by abnormally low levels of neutrophils in the circulating blood. Neutrophils are a specific kind of white blood cell that help prevent and fight infections. The most common reason that cancer patients experience neutropenia is as a side effect of chemotherapy. Chemotherapy-induced neutropenia increases a patient's risk of infection and disrupts cancer treatment.

In a particular embodiment of the present invention, the immunocompromised subject to be vaccinated has a CD4+ cell count below 500/mm³, or CD4+ cell count below 300/mm³, or CD4+ cell count below 200/mm³, CD4+ cell count below 100/mm³, CD4+ cell count below 75/mm³, or CD4+ cell count below 50/mm³.

CD4 cell tests are normally reported as the number of cells in mm³. Normal CD4 counts are between 500 and 1600, and CD8 counts are between 375 and 1100. CD4 counts drop dramatically in people with HIV.

In an embodiment of the invention, any of the immunocompromised subject disclosed herein is a human male or a human female.

The amount of a conjugate in a composition is generally calculated based on total polysaccharide, conjugated and non-conjugated for that conjugate. For example, a conjugate with 20% free polysaccharide will have about 80 µg of conjugated polysaccharide and about 20 µg of non-conjugated polysaccharide in a 100 µg polysaccharide dose. The protein contribution to the conjugate is usually not considered when calculating the dose of a conjugate. Generally, each dose will comprise 0.1 to 100 µg of polysaccharide, particularly 0.1 to 10 µg, and more particularly 1 to 10 µg and more particularly 1 to 5 µg. Preferably each dose will comprise about 1.1, 2, 2.2, 3, 3.3, 4, 4.4 µg of polysaccharide.
Optimal amounts of components for a particular immunogenic composition or vaccine can be ascertained by standard studies involving observation of appropriate immune responses in subjects. Following an initial vaccination, subjects can receive one or several booster immunizations adequately spaced.

The effectiveness of an antigen as an immunogen, can be measured either by proliferation assays, by cytolytic assays, such as chromium release assays to measure the ability of a T-cell to lyse its specific target cell, or by measuring the levels of B-cell activity by measuring the levels of circulating antibodies specific for the antigen in serum. An immune response may also be detected by measuring the serum levels of antigen specific antibody induced following administration of the antigen, and more specifically, by measuring the ability of the antibodies so induced to enhance the opsonophagocytic ability of particular white blood cells, as described herein. The level of protection of the immune response may be measured by challenging the immunized host with the antigen that has been administered. For example, if the antigen to which an immune response is desired is a bacterium, the level of protection induced by the immunogenic amount of the antigen is measured by detecting the percent survival or the percent mortality after challenge of the animals with the bacterial cells. In one embodiment, the amount of protection may be measured by measuring at least one symptom associated with the bacterial infection, e.g., a fever associated with the infection. The amount of each of the antigens in the multi-antigen or multi-component vaccine or immunogenic compositions will vary with respect to each of the other components and can be determined by methods known to the skilled artisan. Such methods would include procedures for measuring immunogenicity and/ or *in vivo* efficacy.

The disclosure further provides antibodies and antibody compositions which bind specifically and selectively to the capsular polysaccharides or immunogenic conjugates of the present disclosure. In some embodiments, antibodies are generated upon administration to a subject of the capsular polysaccharides or immunogenic conjugates of the present disclosure. In some embodiments, the disclosure provides purified or isolated antibodies directed against one or more of the capsular polysaccharides or immunogenic conjugates of the present disclosure. In some embodiments, the antibodies of the present disclosure are functional as measured by killing bacteria in either an animal efficacy model or via an opsonophagocytic killing assay. In some embodiments, the antibodies of the disclosure confer passive immunity to a subject. The present disclosure further provides polynucleotide molecules encoding an antibody or antibody fragment of the disclosure, and a cell, cell line (such as hybridoma cells or other engineered cell lines for recombinant production of antibodies) or a transgenic animal that produces an antibody or antibody composition of the disclosure, using techniques well-known to those of skill in the art.

### Examples

### Example 1: Preparation of isolated Streptococcus pneumoniae serotype 15B capsular polysaccharide

### 1.1 Fermentation and Purification

Serotype 15B capsular polysaccharides can be obtained directly from bacteria using isolation procedures known to one of ordinary skill in the art (see for example methods disclosed U.S. Patent App. Pub. Nos. 20060228380, 20060228381, 20070184071, 20070184072, 20070231340, and 20080102498 or WO2008118752). The serotype 15B *Streptococcus pneumonia* were grown in a seed bottle and then transferred to a seed fermentor. Once the targeted optical density was reached, the cells were transferred to a production fermentor. The fermentation was broth was inactivated by the addition of N-lauroyl sarcosine and purified by ultrafiltration and diafiltration.

The purified *Streptococcus pneumoniae* serotype 15B polysaccharide was then sized by high pressure homogenization using a PANDA 2K homogenizer ® (GEA Niro Soavi) to produce the isolated *Streptococcus pneumoniae* serotype 15B polysaccharide.

Preferably, the isolated *Streptococcus pneumoniae* serotype 15B capsular polysaccharide obtained by the above process comprises at least 0.6 mM acetate per mM of serotype 15B capsular polysaccharide and has a molecular weight between 50kDa and 500kDa, preferably 150 to 350kDa.

### 1.2 Oxidation of Isolated Streptococcus pneumoniae serotype 15B capsular polysaccharide

Polysaccharide oxidation was carried out in 100 mM potassium phosphate buffer (pH 6.0 ± 0.2) by sequential addition of calculated amount of 500 mM potassium phosphate buffer (pH 6.0) and WFI to give final polysaccharide concentration of 2.0 g/L. If required, the reaction pH was adjusted to pH 6.0, approximately. After pH adjustment, the reaction temperature was adjusted to 23 ± 2 °C. Oxidation was initiated by the addition of approximately 0.25 molar equivalents of sodium periodate. The oxidation reaction was performed at 23 ± 2 °C during 16 hrs, approximately.

Concentration and diafiltration of the activated polysaccharide was carried out using 10K MWCO ultrafiltration cassettes. Diafiltration was performed against 20-fold diavolumes of WFI. The purified activated polysaccharide was then stored at 5 ± 3°C. The purified activated saccharide was characterized inter alia by (i) saccharide concentration by colorimetric assay; (ii) aldehyde concentration by colorimetric assay; (iii) Degree of Oxidation (iv) Molecular Weight by SEC-MALLS and (v) presence of O-acetyl and glycerol.

SEC-MALLS is used for the determination of the molecular weight of polysaccharides and polysaccharide-protein conjugates. SEC is used to separate the polysaccharides by hydrodynamic volume. Refractive index (RI) and multi-angle laser light scattering (MALLS) detectors are used for the determination of the molecular weight. When light interacts with matter, it scatters and the amount of scattered light is related to the concentration, the square of the dn/dc (the specific refractive index increments), and the molar mass of the matter. The molecular weight measurement is calculated based on the readings from the scattered light signal from the MALLS detector and the concentration signal from the RI detector.

The degree of oxidation (DO = moles of sugar repeat unit / moles of aldehyde) of the activated polysaccharide was determined as follows:
The moles of sugar repeat unit is determined by various colorimetric methods, example by using Anthrone method. By the Anthrone mthod, the polysaccharide is first broken down to monosaccharides by the action of sulfuric acid and heat. The Anthrone reagent reacts with the hexoses to form a yellow-green colored complex whose absorbance is read spectrophotometrically at 625nm. Within the range of the assay, the absorbance is directly proportional to the amount of hexose present.
The moles of aldehyde also is determined simultaneously, using MBTH colorimetric method. The MBTH assay involves the formation of an azine compound by reacting aldehyde groups (from a given sample) with a 3-methyl-2-benzothiazolone hydrazone (MBTH assay reagent). The excess 3-methyl-2-benzothiazolone hydrazone oxidizes to form a reactive cation. The reactive cation and the azine react to form a blue chromophore. The formed chromophore is then read spectroscopically at 650 nm.

Preferably, the activated *Streptococcus pneumoniae* serotype 15B capsular polysaccharide obtained by the above process comprises at least 0.6 mM acetate per mM of serotype 15B capsular polysaccharide and has a molecular weight between 50kDa and 500kDa, preferably 100 to 250kDa.

### 1.3 Conjugation of activated Streptococcus pneumoniae serotype 15B capsular polysaccharide with CRM₁₉₇

The conjugation process consists of the following steps:
a) Compounding with sucrose excipient and lyophilization
b) Reconstitution of the lyophilized activated polysaccharide and CRM₁₉₇
c) Conjugation of activated polysaccharide to CRM₁₉₇ and capping
d) Purification of the conjugate

### a) Compounding with Sucrose excipient, and Lyophilization

The activated polysaccharide was compounded with sucrose to a ratio of 25 grams of sucrose per gram of activated polysaccharide. The bottle of compounded mixture was then lyophilized. Following lyophilization, bottles containing lyophilized activated polysaccharide were stored at -20 ± 5°C. Calculated amount of CRM₁₉₇ protein was shell-frozen and lyophilized separately. Lyophilized CRM₁₉₇ was stored at -20 ± 5°C.

### b) Reconstitution of Lyophilized Activated Polysaccharide and CRM₁₉₇ Protein

Lyophilized activated polysaccharide was reconstituted in anhydrous dimethyl sulfoxide (DMSO). Upon complete dissolution of polysaccharide, an equal amount of anhydrous DMSO was added to lyophilized CRM₁₉₇ for reconstitution.

### c) Conjugation and Capping

Reconstituted activated polysaccharide was combined with reconstituted CRM₁₉₇ in the reaction vessel (input ratio: 0.8:1), followed by mixing thoroughly to obtain a clear solution before initiating the conjugation with sodium cyanoborohydride. The final polysaccharide concentration in reaction solution is approximately 1 g/L. Conjugation was initiated by adding 1.0 - 1.5 MEq of sodium cyanoborohydride to the reaction mixture and was incubated at 23 ± 2 °C for 40-48 hrs. Conjugation reaction was terminated by adding 2 MEq of sodium borohydride (NaBH₄) to cap unreacted aldehydes. This capping reaction continued at 23 ± 2°C for 3 ± 1 hrs.

### d) Purification of the conjugate

The conjugate solution was diluted 1:10 with chilled 5 mM succinate-0.9% saline (pH 6.0) in preparation for purification by tangential flow filtration using 100-300K MWCO membranes. The diluted conjugate solution was passed through a 5 µm filter and diafiltration was performed using 5 mM succinate-0.9% saline (pH 6.0) as the medium. After the diafiltration was completed, the conjugate retentate was transferred through a 0.22µm filter.
The conjugate was diluted further with 5 mM succinate / 0.9% saline (pH 6), to a target saccharide concentration of approximately 0.5 mg/mL. Final 0.22µm filtration step was completed to obtain the immunogenic conjugate.

Preferably, the conjugate obtained by the above process comprises at least 0.6 mM acetate per mM of serotype 15B capsular polysaccharide, has a molecular weight between 3000 and 20000kDa and has a degree of conjugation between 2 and 6.

### Example 2: Characterization of immunogenic conjugate comprising Streptococcus pneumoniae serotype 15B capsular polysaccharide covalently linked to a CRM₁₉₇

Conjugate 1 was prepared by the process disclosed in example 1. Conjugates 2 and 3 were prepared by a similar process using different amount of oxidizing agent. Conjugate 4 was prepared by a similar process except that the purified serotype 15B capsular polysaccharide was not sized and was activated to a lower DO (higher oxidation level) and the conjugation was performed in aqueous medium. Conjugate 5 was prepared by a similar process except that the purified serotype 15B capsular polysaccharide was sized by chemical hydrolysis and the conjugation was performed in aqueous medium. Conjugates 6 and 7 were prepared by a similar process except that the purified serotype 15B capsular polysaccharide was not sized. The obtained conjugates were characterized and the results are summarized in Table 1.

**Table 1: Streptococcus pneumoniae serotype 15B capsular polysaccharide-CRM₁₉₇ conjugates**

| Conjugate | 1 | 2 | 3 | 4 | 5 | 6 | 7 |
|---|---|---|---|---|---|---|---|
| Polysaccharide | Sized | Sized | Sized | Native | Hydrolyzed | Native | Native |
| O-Acetylation; Polysaccharide (µmol acetate/µmol poly) | 0.69 | 0.69 | 0.69 | 1.01 | 0.66 | 0.76 | NA |
| Solvent medium | DMSO | DMSO | DMSO | Aqueous | Aqueous | DMSO | DMSO |
| Activated Polysaccharide DO | 11.4 | 5.8 | 9.7 | 4.8 | 8.8 | 5 | 12 |
| Activated Polysaccharide MW | 196KDa | 218KDa | 235KDa | 435 KDa | 270KDa | 431KDa | 460KDa |
| Yield (%) | 87.2 | 64 | 63.7 | 96.2 | 78.8 | 24.2 | 26.2 |
| Saccharide Protein Ratio | 0.68 | 0.65 | 0.71 | 1.22 | 1.29 | 0.9 | 1.5 |
| Free Saccharide (%) | < 5 | < 5 | 6.1 | 18.1 | 14.2 | 8.8 | 18 |
| Conjugate MW, SEC-MALLS | 6190KDa | 7090KDa | 7937KDa | 1766KDa | 1029KDa | 6293KDa | 4466KDa |
| O-Acetylation, Conjugate (µmol acetate/µmol poly) | 0.68 | 0.7 | 0.68 | 0.61 | 0.44 | 0.85 | NA |
| < 0.3 Kd (%), SEC | NA | 73 | NA | NA | 62 | NA | NA |
| Degree of Conj (AAA); Modified Lys | 3.7 | 3.9 | 4.1 | NA | 3.4 | NA | NA |
| % O-Acetyl Retained in Conjugate | 99% | 100% | 99.5% | 60% | 67% | 100% | NA |

The percentage of free polysaccharide is measured by a procedure utilizing aluminium hydroxide gel to bind protein and covalently bound saccharide for removal by centrifugation. Samples are mixed with phosphate buffered aluminium hydroxide gel and centrifuged. Bound saccharide is pelleted with the gel and free saccharide remains in the supernatant. The resulting supernatant and controls samples are quantitated by appropriate colorimetric assays to determine the percentage of free saccharide and to confirm sufficient removal of protein and recovery of saccharide.

For the Amino Acid analysis the polysaccharide-protein sample is first hydrolyzed into its individual components as free amino acids, using 6N hydrochloric acid (HCI) hydrolysis under vacuum and heat (160° C for 15 minutes). After hydrolysis, the samples are analyzed using Amino Acid Analyzer. The individual amino acids are separated through ion exchange chromatography using a step gradient of sodium citrate buffer with temperature and flow rate changes. After separation, the amount of each amino acid residual is quantitatively determined using a postcolumn ninhydrin coupling detection system. In this system, the ninhydrin is mixed with the column eluate in the postcolumn reactor system and the mixture passed into the photometer. The reaction of ninhydrin with eluated amino acids yields a purple compound that absorbs maximally at 570 nm. This absorbance is a linear response (function) of the amount of α-amino groups present and this reaction provides quantitative colorimetric assay for all organic compounds with α-amino groups. In the reaction with imino acids such as proline and hydroxylproline, which do not have free amino group, a bright yellow compound is generated and monitored at 440 nm. The peak areas for each amino acid are calculated using both 570 and 440 nm wavelength outputs.

The yield is calculated as follows: (amount of polysaccharide in the conjugate x100) / amount of activated polysaccharide.

Conjugates (4 and 5) generated using in aqueous medium demonstrated significant loss in O-acetyl levels. Conjugates generated in DMSO solvent, using native polysaccharide without MW sizing (6 and 7) did not demonstrate loss in O-acetyl levels. However, the conjugate yields were very poor in addition to poor filterability characteristics. Conjugates generated in DMSO using polysaccharides that were sized by high pressure homogenization (1, 2 and 3) had high yield and better filterability characteristics with significant preservation of O-acetyl levels. These conjugates also had very low levels of free polysaccharides.

### Example 3: Opsonophagocytic activity (OPA) assay

The immunogenicity of the conjugates of the invention can be assessed using the opsonophagocytic assay (OPA) described below.

Groups of 30 6-7 week old female Swiss Webster mice were immunized with 0.001 µg, 0.01 µg, or 0.1 µg of test conjugates via the subcutaneous route on week 0. The mice were boosted with the same dose of conjugate on week 3 and then bled at week 4. Serotype-specific OPAs were performed on week 4 sera samples.

OPAs are used to measure functional antibodies in murine sera specific for *S. pneumoniae* serotype 15B. Test serum is set up in assay reactions that measure the ability of capsular polysaccharide specific immunoglobulin to opsonize bacteria, trigger complement deposition, thereby facilitating phagocytosis and killing of bacteria by phagocytes. The OPA titer is defined as the reciprocal dilution that results in a 50% reduction in bacterial count over control wells without test serum. The OPA titer is interpolated from the two dilutions that encompass this 50% killing cut-off.

OPA procedures were based on methods described in Hu et al., Clin Diagn Lab Immunol 2005;12(February (2)):287-95 with the following modifications. Test serum was serially diluted 2.5-fold and added to microtiter assay plates. Live serotype 15B target bacteria were added to the wells and the plates were shaken at 37°C for 30 minutes. Differentiated HL-60 cells (phagocytes) and baby rabbit serum (3- to 4-week old, Pel-Freez®, 6.25% final concentration) were added to the wells, and the plates were shaken at 37°C for 45 minutes. To terminate the reaction, 80 µL of 0.9% NaCl was added to all wells, mixed, and a 10µL aliquot were transferred to the wells of MultiScreen HTS HV filter plates (Millipore®) containing 200 µL of water. Liquid was filtered through the plates under vacuum, and 150 µL of HySoy medium was added to each well and filtered through. The filter plates were then incubated at 37°C, 5% CO₂ overnight and were then fixed with Destain Solution (Bio-Rad). The plates were then stained with Coomassie Blue and destained once. Colonies were imaged and enumerated on a Cellular Technology Limited (CTL) ImmunoSpot Analyzer®. Raw colony counts were used to plot kill curves and calculate OPA titers.

The immunogenicity of conjugates 1 and 2 has been tested according to the above mentioned assay. One additional conjugate and an unconjugated native *S. pneumoniae* serotype 15B capsular polysaccharide (unconjugated PS) were also tested in the same assay:
Conjugate 9 was prepared by conjugation of native (i.e not sized) serotype 15B capsular polysaccharide to CRM₁₉₇ by reductive amination in aqueous solution.
The results are shown in table 2.

**Table 2: OPA Titers of Animal Testing**

| OPA GMT (Geometric Mean antibody Titer) (95% CI) | | | |
|---|---|---|---|
| | 0.001 µg | 0.01 µg | 0.1 µg |
| Conjugate 1 | 485 (413, 569) | 804 (565, 1145) | 1563 (1048, 2330) |
| Conjugate 2 | 556 (438, 707) | 871 (609, 1247) | 1672 (1054, 2651) |
| Conjugate 9 | 395 (329, 475) | 856 (627, 1168) | 1802 (1108, 2930) |
| Unconjugated PS | - | - | 698 (466, 1045) |

As shown in the above table, conjugates 1 and 2, when administered to mice, generate antibodies capable of opsonizing serotype 15B *S. pneumoniae,* triggering complement deposition, thereby facilitating phagocytosis and killing of bacteria by phagocytes. In addition, despite their lower molecular weight, they also exhibited similar level of immunogenicity as compared to conjugate 9 which has not been sized.

### Example 4: Cross-functional OPA responses between serotype 15B and serotype 15C

Pneumococcal serogroup 15 includes four structurally-related serotypes: 15A, 15B, 15C, and 15F. Serotypes 15B and 15C are undistinguishable by genetic typing techniques and have similar capsular polysaccharide (PS) composition, except that the 15B-PS is the O-acetylated variant of 15C-PS. To understand whether anti-capsular PS antibodies for serotype 15B are functionally cross-reacting with serotype 15C, 10 rabbits were immunized with PCV16v and PCV20v vaccines both containing an immunogenic conjugate comprising *Streptococcus pneumoniae* serotype 15B capsular polysaccharide covalently linked to CRM₁₉₇ as disclosed herein as part of their formulation. Sera from pre- and post-vaccination were tested in OPA assays against serotypes 15B and 15C target pneumococcal strains.

Of the 10 rabbits from each group, 100% had OPA response to serotype 15B following immunization with a serotype 15B conjugate. Of these same samples, 100% had OPA response to serotype 15C as well (Table 1 and Table 2). Low OPA titers were observed in prevaccination sera in 15C OPA. However, over 10-fold GMT OPA titer increase with post vaccination sera compared to pre vaccination demonstrated that the immunogenic conjugates of the invention induces the formation of antibodies capable of killing serotype 15B and 15C *Streptococcus* pneumonia in an OPA.

PCV16v is a 16-valent conjugates composition comprising glycoconjugates from *S. pneumoniae* serotypes 1, 3, 4, 5, 6A, 6B, 7F, 9V, 14, 15B, 18C, 19A, 19F, 22F, 23F and 33F (16vPnC) all individualy conjugated to CRM₁₉₇.

PCV20v is a 20 valent conjugates composition comprising glycoconjugates from *S. pneumoniae* serotypes 1, 3, 4, 5, 6A, 6B, 7F, 8, 9V, 10A, 11A, 12F, 14, 15B, 18C, 19A, 19F, 22F, 23F and 33F (20vPnC) all individualy conjugated to CRM₁₉₇.

**Table 1. OPA Titers Against serotypes 15B and 15C strains in Rabbit Sera Pre and Post vaccination with PCV16v**

| Animal | 15B OPA | | 15C OPA | |
|---|---|---|---|---|
| | wk0 | wk4 | wk0 | wk4 |
| 1 | 4 | 4129 | 50 | 2524 |
| 2 | 4 | 1645 | 182 | 472 |
| 3 | 4 | 1131 | 126 | 818 |
| 4 | 4 | 3199 | 50 | 1189 |
| 5 | 4 | 2664 | 36 | 727 |
| 6 | 4 | 4589 | 68 | 2492 |
| 7 | 11 | 3601 | 169 | 1137 |
| 8 | 4 | 1838 | 165 | 672 |
| 9 | 4 | 1334 | 98 | 528 |
| 10 | 4 | 1108 | 204 | 2425 |
| **GMT** | **4** | **2222** | **98** | **1075** |

**Table 2. OPA Titers Against serotypes 15B and 15C strains in Rabbit Sera Pre and Post vaccination with PCV20v**

| Animal | 15B OPA | | 15C OPA | |
|---|---|---|---|---|
| | wk0 | wk4 | wk0 | wk4 |
| 1 | 4 | 3784 | indeterminable* | 2353 |
| 2 | 4 | 862 | 480 | 938 |
| 3 | 4 | 3056 | 69 | 1497 |
| 4 | 4 | 1948 | indeterminable* | 1316 |
| 5 | 4 | 2360 | 4 | 4665 |
| 6 | 4 | 1594 | indeterminable* | 1835 |
| 7 | 4 | 4943 | 172 | 4085 |
| 8 | 4 | 2419 | 117 | 1458 |
| 9 | 4 | 1245 | indeterminable* | 527 |
| 10 | 4 | 616 | indeterminable* | 545 |
| **GMT** | **4** | **1917** | **77** | **1515** |

| | | | | |
|---|---|---|---|---|
| * Titer cannot be determined due to bad killing curves | | | | |

## Claims

1. A process for the preparation of an immunogenic conjugate comprising *Streptococcus pneumoniae* serotype 15B capsular polysaccharide covalently linked to a carrier protein, the process comprising the steps of:
(a) compounding an activated serotype 15B capsular polysaccharide with a carrier protein, wherein said activated polysaccharide is obtained by a process comprising the steps of reacting an isolated *Streptococcus pneumoniae* serotype 15B capsular polysaccharide comprising at least 0.6 mM acetate per mM of said serotype 15B capsular polysaccharide, with an oxidizing agent, wherein said activated polysaccharide has a molecular weight between 100 and 300kDa; and
(b) reacting the compounded, activated polysaccharide and carrier protein with a reducing agent to form a serotype 15B capsular polysaccharide-carrier protein conjugate;
wherein step (a) and step (b) are carried out in DMSO.

2. The process according to claim 1 wherein the carrier protein is CRM₁₉₇.

3. The process according to any one of claims 1 to 2 wherein the concentration of activated serotype 15B capsular polysaccharide in step (b) is between 0.1 and 10 mg/mL, 0.5 and 5 mg/mL or 0.5 and 2 mg/mL.

4. The process according to any one of claims 1 to 3, wherein the initial input ratio of activated serotype 15B capsular polysaccharide to carrier protein is between 5:1 and 0.1:1, 2:1 and 0.1:1, 2:1 and 1:1, 1.5:1 and 1:1, 0.1:1 and 1:1, 0.3:1 and 1:1, 0.6:1 and 1:1, or 0.6:1 and 1.5:1.

5. The process according to any one of claims 1 to 4, wherein in step (b), the activated polysaccharide is reacted with between 1 and 2 molar equivalent of sodium cyanoborohydride during 40 to 50 hours at a temperature between 20 to 26°C.

6. The process according to any one of claims 1 to 5 wherein said process comprises the additional following step:
(c) capping unreacted aldehyde by addition of NaBH₄.

7. The process according to any one of claims 1 to 6, wherein said oxidizing agent is periodate.

8. The process according to any one of claims 1 to 6, wherein said oxidizing agent is sodium periodate.

9. The process according to any one of claims 1 to 6, wherein said oxidizing agent is sodium metaperiodate.

10. The process according to any one of claims 1 to 9 wherein the yield of the conjugation step (b) is greater than 50%.

11. The process according to any one of claims 1 to 10 wherein said process further comprises the step of formulating the conjugate in a multivalent vaccine.

12. An immunogenic conjugate obtained by the process of any one of claims 1 to 11.

13. The immunogenic conjugate according to claim 12 wherein said immunogenic conjugate comprises less than 40% of free serotype 15B capsular polysaccharide compared to the total amount of serotype 15B capsular polysaccharide.

14. The immunogenic conjugate according to claim 12 or 13 wherein the immunogenic conjugate has a molecular weight between: 3000 and 20000kDa; 5000 and 10000kDa; 5000 and 20000kDa; 8000 and 20000kDa; 8000 and 16000 KDa; or 10000 and 16000 KDa.

15. The immunogenic conjugate according to any one of claims 12 to 14 wherein the ratio of serotype 15B capsular polysaccharide to carrier protein in the conjugate is between 0.4 and 2.

16. The immunogenic conjugate according to any one of claims 12 to 15 wherein at least 40% of the immunogenic conjugate has a Kd below or equal to 0.3 in a CL-4B column.

17. The immunogenic conjugate according to any one of claims 12 to 16 wherein the degree of conjugation is between: 2 and 15; 2 and 13; 2 and 10; 2 and 8; 2 and 6; 2 and 5, 2 and 4; 3 and 15; 3 and 13; 3 and 10; 3 and 8; 3 and 6; 3 and 5; 3 and 4; 5 and 15; 5 an 10; 8 and 15; 8 and 12; 10 and 15; or 10 and 12.

18. The immunogenic conjugate according to any one of claims 12 to 17 wherein the ratio of mM acetate per mM serotype 15B capsular polysaccharide in the immunogenic conjugate to mM acetate per mM serotype 15B capsular polysaccharide in the activated polysaccharide is at least 0.6, 0.65, 0.7, 0.75, 0.8, 0.85, 0.9, or 0.95, preferably at least 0.7 or at least 0.9.

19. An immunogenic composition comprising an immunogenic conjugate according to any one of claims 12 to 18 and a physiologically acceptable vehicle.

20. A vaccine comprising an immunogenic composition according to claim 19.

21. The immunogenic composition of claim 19, or the vaccine of claim 20, for use in a method of protecting a subject against an infection with serotype 15B *Streptococcus pneumoniae.*

22. The immunogenic composition according to claim 19, or the vaccine of claim 20 for use in a method of treating or preventing a *Streptococcus pneumoniae* infection, disease or condition associated with serotype 15B *Streptococcus pneumoniae* in a subject, said method comprising the step of administering a therapeutically or prophylactically effective amount of immunogenic composition or of said vaccine.

## Patentansprüche

1. Verfahren zur Herstellung eines immunogenen Konjugats, umfassend *Streptococcus* pneumoniae-Kapselpolysaccharid vom Serotyp 15B, das kovalent an ein Trägerprotein gebunden ist, wobei das Verfahren die folgenden Schritte umfasst:
(a) Compoundieren eines aktivierten Kapselpolysaccharids vom Serotyp 15B mit einem Trägerprotein, wobei das aktivierte Polysaccharid durch ein Verfahren erhalten wird, das die Schritte des Umsetzens eines isolierten *Streptococcus pneumoniae-Kap*selpolysaccharids vom Serotyp 15B, das mindestens 0,6 mM Acetat pro mM des Kapselpolysaccharids vom Serotyp 15B umfasst, mit einem Oxidationsmittel umfasst, wobei das aktivierte Polysaccharid ein Molekulargewicht zwischen 100 und 300 kDa aufweist; und
(b) Umsetzen des compoundierten, aktivierten Polysaccharids und Trägerproteins mit einem Reduktionsmittel, um ein Konjugat aus Kapselpolysaccharid vom Serotyp 15B und Trägerprotein zu bilden;
wobei Schritt (a) und Schritt (b) in DMSO durchgeführt werden.

2. Verfahren nach Anspruch 1, wobei das Trägerprotein CRM₁₉₇ ist.

3. Verfahren nach einem der Ansprüche 1 bis 2, wobei die Konzentration des aktivierten Kapselpolysaccharids vom Serotyp 15B in Schritt (b) zwischen 0,1 und 10 mg/ml, 0,5 und 5 mg/ml oder 0,5 und 2 mg/ml liegt.

4. Verfahren nach einem der Ansprüche 1 bis 3, wobei das anfängliche Eingangsverhältnis von aktiviertem Kapselpolysaccharid vom Serotyp 15B zu Trägerprotein zwischen 5:1 und 0, 1:1, 2:1 und 0,1:1, 2:1 und 1:1, 1,5:1 und 1:1, 0,1:1 und 1:1, 0,3:1 und 1:1, 0,6:1 und 1:1 oder 0,6:1 und 1,5:1 liegt.

5. Verfahren nach einem der Ansprüche 1 bis 4, wobei in Schritt (b) das aktivierte Polysaccharid mit zwischen 1 und 2 Moläquivalenten Natriumcyanoborhydrid während 40 bis 50 Stunden bei einer Temperatur zwischen 20 und 26°C umgesetzt wird.

6. Verfahren nach einem der Ansprüche 1 bis 5, wobei das Verfahren den zusätzlichen folgenden Schritt umfasst:
(c) Verkappen von nicht umgesetztem Aldehyd durch Zugabe von NaBH₄.

7. Verfahren nach einem der Ansprüche 1 bis 6, wobei das Oxidationsmittel Periodat ist.

8. Verfahren nach einem der Ansprüche 1 bis 6, wobei das Oxidationsmittel Natriumperiodat ist.

9. Verfahren nach einem der Ansprüche 1 bis 6, wobei das Oxidationsmittel Natriummetaperiodat ist.

10. Verfahren nach einem der Ansprüche 1 bis 9, wobei die Ausbeute des Konjugationsschritts (b) größer als 50% ist.

11. Verfahren nach einem der Ansprüche 1 bis 10, wobei das Verfahren weiterhin den Schritt der Formulierung des Konjugats in einen multivalenten Impfstoff umfasst.

12. Immunogenes Konjugat, erhalten durch das Verfahren nach einem der Ansprüche 1 bis 11.

13. Immunogenes Konjugat nach Anspruch 12, wobei das immunogene Konjugat weniger als 40% freies Kapselpolysaccharid vom Serotyp 15B im Vergleich zur Gesamtmenge an Kapselpolysaccharid vom Serotyp 15B umfasst.

14. Immunogenes Konjugat nach Anspruch 12 oder 13, wobei das immunogene Konjugat ein Molekulargewicht aufweist zwischen: 3000 und 20000 kDa; 5000 und 10000 kDa; 5000 und 20000 kDa; 8000 und 20000 kDa; 8000 und 16000 kDa; oder 10000 und 16000 kDa.

15. Immunogenes Konjugat nach einem der Ansprüche 12 bis 14, wobei das Verhältnis von Kapselpolysaccharid vom Serotyp 15B zu Trägerprotein in dem Konjugat zwischen 0,4 und 2 liegt.

16. Immunogenes Konjugat nach einem der Ansprüche 12 bis 15, wobei mindestens 40% des immunogenen Konjugats einen Kd-Wert unter oder gleich 0,3 in einer CL-4B-Säule aufweist.

17. Immunogenes Konjugat nach einem der Ansprüche 12 bis 16, wobei der Grad der Konjugation zwischen 2 und 15; 2 und 13; 2 und 10; 2 und 8; 2 und 6; 2 und 5, 2 und 4; 3 und 15; 3 und 13; 3 und 10; 3 und 8; 3 und 6; 3 und 5; 3 und 4; 5 und 15; 5 und 10; 8 und 15; 8 und 12; 10 und 15; oder 10 und 12 liegt.

18. Immunogenes Konjugat nach einem der Ansprüche 12 bis 17, wobei das Verhältnis von mM Acetat pro mM Kapselpolysaccharid vom Serotyp 15B im immunogenen Konjugat zu mM Acetat pro mM Kapselpolysaccharid vom Serotyp 15B im aktivierten Polysaccharid mindestens 0,6, 0,65, 0,7, 0,75, 0,8, 0,85, 0,9 oder 0,95, vorzugsweise mindestens 0,7 oder mindestens 0,9 beträgt.

19. Immunogene Zusammensetzung, umfassend ein immunogenes Konjugat nach einem der Ansprüche 12 bis 18 und ein physiologisch annehmbares Vehikel.

20. Impfstoff, umfassend eine immunogene Zusammensetzung nach Anspruch 19.

21. Immunogene Zusammensetzung nach Anspruch 19 oder Impfstoff nach Anspruch 20 zur Verwendung in einem Verfahren zum Schutz eines Subjekts gegen eine Infektion mit *Streptococcus pneumoniae* vom Serotyp 15B.

22. Immunogene Zusammensetzung nach Anspruch 19 oder Impfstoff nach Anspruch 20 zur Verwendung in einem Verfahren zur Behandlung oder Vorbeugung einer/eines *Streptococcus pneumoniae-In*fektion, -Erkrankung oder -Zustands, die/der mit *Streptococcus pneumoniae* vom Serotyp 15B in einem Subjekt assoziiert ist, wobei das Verfahren den Schritt der Verabreichung einer therapeutisch oder prophylaktisch wirksamen Menge der immunogenen Zusammensetzung oder des Impfstoffs umfasst.

## Revendications

1. Procédé pour la préparation d'un conjugué immunogène comprenant un polysaccharide capsulaire de sérotype 15B de *Streptococcus pneumoniae* lié de manière covalente à une protéine support, le procédé comprenant les étapes de :
(a) mélange d'un polysaccharide capsulaire de sérotype 15B activé avec une protéine support, dans lequel ledit polysaccharide activé est obtenu par un procédé comprenant les étapes de mise en réaction d'un polysaccharide capsulaire de sérotype 15B de *Streptococcus pneumoniae* isolé comprenant au moins 0,6 mM d'acétate par mM dudit polysaccharide capsulaire de sérotype 15B, avec un agent oxydant, dans lequel ledit polysaccharide activé a un poids moléculaire entre 100 et 300 kDa ; et
(b) mise en réaction du polysaccharide activé et de la protéine support mélangés avec un agent réducteur afin de former un conjugué polysaccharide capsulaire de sérotype 15B-protéine support ;
dans lequel l'étape (a) et l'étape (b) sont réalisées dans du DMSO.

2. Procédé selon la revendication 1, dans lequel la protéine support est CRM₁₉₇.

3. Procédé selon l'une des revendications 1 à 2, dans lequel la concentration de polysaccharide capsulaire de sérotype 15B activé dans l'étape (b) est entre 0,1 et 10 mg/ml, 0,5 et 5 mg/ml ou 0,5 et 2 mg/ml.

4. Procédé selon l'une des revendications 1 à 3, dans lequel le rapport d'entrée initial de polysaccharide capsulaire de sérotype 15B activé à protéine support est entre 5:1 et 0,1:1, 2:1 et 0,1:1, 2:1 et 1:1, 1,5:1 et 1:1, 0,1:1 et 1:1, 0,3:1 et 1:1, 0,6:1 et 1:1, ou 0,6:1 et 1,5:1.

5. Procédé selon l'une des revendications 1 à 4, dans lequel dans l'étape (b), le polysaccharide activé est mis en réaction avec entre 1 et 2 équivalents molaires de cyanoborohydrure de sodium pendant 40 à 50 heures à une température entre 20 et 26 °C.

6. Procédé selon l'une des revendications 1 à 5, dans lequel ledit procédé comprend l'étape supplémentaire suivante :
(c) le coiffage d'aldéhyde non réagi par addition de NaBH₄.

7. Procédé selon l'une des revendications 1 à 6, dans lequel ledit agent oxydant est du périodate.

8. Procédé selon l'une des revendications 1 à 6, dans lequel ledit agent oxydant est du périodate de sodium.

9. Procédé selon l'une des revendications 1 à 6, dans lequel ledit agent oxydant est du métapériodate de sodium.

10. Procédé selon l'une des revendications 1 à 9, dans lequel le rendement de l'étape de conjugaison (b) est supérieur à 50 %.

11. Procédé selon l'une des revendications 1 à 10, dans lequel ledit procédé comprend en outre l'étape de formulation du conjugué dans un vaccin multivalent.

12. Conjugué immunogène obtenu par le procédé selon l'une des revendications 1 à 11.

13. Conjugué immunogène selon la revendication 12, dans lequel ledit conjugué immunogène comprend moins de 40 % de polysaccharide capsulaire de sérotype 15B libre en comparaison à la quantité totale de polysaccharide capsulaire de sérotype 15B.

14. Conjugué immunogène selon la revendication 12 ou 13, dans lequel le conjugué immunogène a un poids moléculaire entre : 3000 et 20 000 kDa ; 5000 et 10 000 kDa ; 5000 et 20 000 kDa ; 8000 et 20 000 kDa ; 8000 et 16 000 KDa ; ou 10 000 et 16 000 KDa.

15. Conjugué immunogène selon l'une des revendications 12 à 14, dans lequel le rapport de polysaccharide capsulaire de sérotype 15B à protéine support dans le conjugué est entre 0,4 et 2.

16. Conjugué immunogène selon l'une des revendications 12 à 15, dans lequel au moins 40 % du conjugué immunogène a un Kd inférieur ou égal à 0,3 dans une colonne CL-4B.

17. Conjugué immunogène selon l'une des revendications 12 à 16, dans lequel le degré de conjugaison est entre : 2 et 15 ; 2 et 13 ; 2 et 10 ; 2 et 8 ; 2 et 6 ; 2 et 5, 2 et 4 ; 3 et 15 ; 3 et 13 ; 3 et 10 ; 3 et 8 ; 3 et 6 ; 3 et 5 ; 3 et 4 ; 5 et 15 ; 5 et 10 ; 8 et 15 ; 8 et 12 ; 10 et 15 ; ou 10 et 12.

18. Conjugué immunogène selon l'une des revendications 12 à 17, dans lequel le rapport de mM d'acétate par mM de polysaccharide capsulaire de sérotype 15B dans le conjugué immunogène à mM d'acétate par mM de polysaccharide capsulaire de sérotype 15B dans le polysaccharide activé est d'au moins 0,6, 0,65, 0,7, 0,75, 0,8, 0,85, 0,9 ou 0,95, de préférence d'au moins 0,7 ou d'au moins 0,9.

19. Composition immunogène comprenant un conjugué immunogène selon l'une des revendications 12 à 18 et un véhicule physiologiquement acceptable.

20. Vaccin comprenant une composition immunogène selon la revendication 19.

21. Composition immunogène selon la revendication 19, ou vaccin selon la revendication 20, pour une utilisation dans une méthode de protection d'un sujet contre une infection avec *Streptococcus pneumoniae* de sérotype 15B.

22. Composition immunogène selon la revendication 19, ou vaccin selon la revendication 20, pour une utilisation dans une méthode de traitement ou de prévention d'une infection avec *Streptococcus pneumoniae,* d'une maladie ou d'un trouble associé à *Streptococcus pneumoniae* de sérotype 15B chez un sujet, ladite méthode comprenant l'étape d'administration d'une quantité thérapeutiquement ou prophylactiquement efficace de composition immunogène ou dudit vaccin.
